(19) Europäisches Patentamt
European Patent Office
Office européen des brevets

(11) **EP 3 745 957 B1**

(12) **EUROPEAN PATENT SPECIFICATION**

(45) Date of publication and mention
of the grant of the patent:
**27.04.2022 Bulletin 2022/17**

(21) Application number: **19714851.3**

(22) Date of filing: **29.01.2019**

(51) International Patent Classification (IPC):
***A61B 5/1455*** (2006.01)

(52) Cooperative Patent Classification (CPC):
**A61B 5/14555**

(86) International application number:
**PCT/NL2019/050050**

(87) International publication number:
**WO 2019/147135 (01.08.2019 Gazette 2019/31)**

(54) **RETINAL OXIMETRY WITH IMPROVED ACCURACY**

RETINALE OXIMETRIE MIT VERBESSERTER GENAUIGKEIT

OXYMÉTRIE RÉTINIENNE À PRÉCISION AMÉLIORÉE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **29.01.2018 NL 2020341**

(43) Date of publication of application:
**09.12.2020 Bulletin 2020/50**

(73) Proprietor: **Stichting VU
1081 HV Amsterdam (NL)**

(72) Inventors:
• **DAMODARAN, Mathi**
**1081 HV Amsterdam (NL)**
• **AMELINK, Arjen**
**1081 HV Amsterdam (NL)**
• **DE BOER, Johannes Fitzgerald**
**1081 HV Amsterdam (NL)**

(74) Representative: **V.O.
P.O. Box 87930
2508 DH Den Haag (NL)**

(56) References cited:
WO-A1-00/06017    US-A- 5 318 022
US-A- 5 776 060    US-A1- 2007 219 439

• BLAIR NORMAN P ET AL: "Retinal Oximetry and Vessel Diameter Measurements With a Commercially Available Scanning Laser Ophthalmoscope in Diabetic Retinopathy.", INVESTIGATIVE OPHTHALMOLOGY & VISUAL SCIENCE 01 10 2017, vol. 58, no. 12, 1 October 2017 (2017-10-01), pages 5556-5563, XP002784885, ISSN: 1552-5783
• HAMMER MARTIN ET AL: "Retinal vessel oximetry-calibration, compensation for vessel diameter and fundus pigmentation, and reproducibility.", JOURNAL OF BIOMEDICAL OPTICS, vol. 13, no. 5, September 2008 (2008-09), page 54015, XP002784886, ISSN: 1083-3668

**Description**

TECHNICAL FIELD AND BACKGROUND

The present disclosure relates to methods and systems for measuring retinal oxygenation.

Haemoglobin oxygen saturation in the blood flowing through the retinal vasculature is an important physiological parameter involved in the pathophysiology of numerous retinal diseases. For example, measuring the retinal blood oxygen saturation may enable monitoring of the development of hypoxia and possibly allow preventing the loss of retinal tissue due to vasoproliferation through timely therapeutic interventions. Furthermore, although damage occurring to the retina in advanced stages of many retinal diseases associated with hypoxia may be established, oxygenation measurements could also aid in understanding the onset or presence of systemic diseases.

Various imaging modalities can be employed to estimate oxygen saturation including fundus camera, hyperspectral imaging, scanning laser ophthalmoscope, adaptive optics SLO and Optical Coherence Tomography. These non-invasive techniques may involve recording the light from the fundus and measuring the amount of light absorbed by the blood flowing through the retinal vessels at different wavelengths. US patent number 5,776,060 relates to a method and apparatus for measuring blood oxygen saturation within a retinal vessel.

Clinical systems are typically based on fundus camera technology, which is snapshot imaging of the retina using a broadband light source. In detection, light is filtered out from a specific band of wavelengths and saturation estimation is performed using an empirical algorithm that utilizes the intensities of the image inside and outside the blood vessels. Unfortunately, the use of broadband light may expose the eye to an unnecessary high intensity.

Scanning laser ophthalmoscope (SLO) based oximetry has potential advantages compared to a conventional fundus camera-based method. For example, an important factor for clinically relevant retinal oximetry is to be able to determine blood oxygen saturation in small retinal vessels - capillaries, venules and arterioles. It is in these microvessels that the oxygen saturation may particularly decrease in response to increased metabolic demand or decreased oxygen delivery capacity. The larger retinal vessels (>100 $\mu$m) may be less sensitive to changes in tissue metabolic demand or to microvascular dysfunction, be less reliable in detecting early hypoxia markers. An SLO can offer superior resolution compared to a fundus camera thereby allowing imaging blood vessels < 50 $\mu$m in high contrast. While SLO may use selected wavelengths, the selection may not be optimal to distinguish retinal oxygenation. Also the signals may be inconsistent for different vessel geometries.

It is thus desired to improve selection of wavelengths for optimizing the measurement of retinal oxygenation particularly in variable vessel geometries.

SUMMARY

The inventors find that an important factor which hitherto has not been considered in retinal oximetry is the fact that haemoglobin is not distributed homogeneously in the tissue, but confined to discrete blood vessels. The effective absorption coefficients of oxy- and deoxyhaemoglobin measured in blood packed in the vessels is different compared to homogenous volumes of haemoglobin freely suspended in a solution. This is akin to an effect which is referred to as "pigment packaging". See for example Finlay et al. [Opt. Lett. 29, 965-967 (2004)], Amelink et $\alpha$/. [Opt. Lett. 34, 1525-1527 (2009)], Rajaram, et al. [Lasers Surg. Med. 42, 680-688 (2010)].

In summary, the pigment packing factor changes the measured spectrum between a homogeneously distributed and a discretely distributed absorber. Without being bound by theory, the effect can be understood as follows. For a homogeneously distributed absorber in a scattering medium, the fluence (isotropic local light intensity) is homogeneous. However, for discretely distributed absorbers (such as densely packed haemoglobin in a blood vessels), the fluence may not be constant in the blood vessel. Since the absorption is very strong, light can be absorbed before it reaches the core of the blood vessel. The fluence may thus becomes a function of the distance from the blood vessel core. As the fluence is lower in the blood vessel core, also less light will be absorbed by the absorber in the core of the vessel. It is clear now that absorbers at the edge of the blood vessel absorb much more light per absorber than absorbers in the core of the blood vessel. This effect can become stronger as the absorption coefficient of the absorber increases, which may cause a spectral dependence of this effect related to the spectral absorption coefficient of the absorber. The reduced absorption by the absorbers in the core of the blood vessel can be considered as a reduction on the concentration of absorbers. The effect can be taken into account for example by defining an effective fluence in the blood vessel, defined by the average fluence in the blood vessel ($\varphi_{avg}$), over the fluence outside of the blood vessel ($\varphi_0$), e.g. expressed as $C_{diff} = \varphi_{avg} / \varphi_0$. Also other or similar approximations can be used, e.g. as described herein. The inventors find that including the pigment packing effect in retinal oximetry algorithms can increase the accuracy of the saturation estimation. Also described herein are procedures to choose optimum wavelengths for oximetry using a detailed error analysis of the modified oximetry equation based on the pigment packaging effect.

In accordance with these and other aspects, the present disclosure provides a method for measuring retinal oxygenation. A retinal tissue with blood vessels can be illuminated by light. Preferably, the light essentially or exclusively consists of discrete wavelengths which may include narrow wavelength bands preferably less than ten nanometer, more preferably less than five nanometer, or even less than one nanometer. By using discrete wavelengths or narrow wavelength bands instead of broadband light, the total intensity may be lower since only specific wavelengths may be needed for optimizing signals as described herein. As described herein preferably, the light consists of a selection of at least two, preferably three, or possibly more discrete wavelengths or narrow wavelength bands. In some embodiments, the dependence of the intensities on the oxygenation is modelled based on predetermined spectra of oxy- and deoxyhaemoglobin. Preferably, an absorption difference between the spectra is different for each of the selected wavelengths. In this way the measurements may be linearly independent which can be used to eliminate parameters and/or improve fitting constraints.

The resulting backscattered reflection intensities from the retina can be measured for collecting a plurality of measurements. The measurements may be spectrally resolved for the selection of discrete wavelengths. In other words, the intensity can be determined as a function of each wavelength. Furthermore, the measurements are preferably position-resolved. In particular, the intensities can be determined for several positions including a blood vessel location coinciding with a selected blood vessel and a tissue location coinciding with the retinal tissue without blood vessel. Multiple measurements representing the same location can be averaged over adjacent positions and/or over time. For example, multiple measurements at different locations along or near a centre of the blood vessel can be averaged. For example, multiple measurements at different locations adjacent on one or both sides of the blood vessel can averaged.

The oxygenation can be calculated based on the measurements. In a preferred embodiment, the calculation includes a model describing a dependence of the intensities on the oxygenation of blood in the selected blood vessel. More preferably, the model also includes a dependence of the measured intensity on a size of the selected blood vessel, e.g. radius, diameter or other cross-section size. Preferably, the model includes on or more analytical functions to calculate the dependencies. The parameters can be calculated or fitted based on a combination of the measurements and the modelled dependencies of the measurements on the parameters. It will be appreciated that an analytical model can provide greater accuracy over various combinations of multivariate data having many parameters such as variable absorption spectra, vessel geometry, and possibly other parameters or inputs such as scattering function et cetera. Additionally, the analytical model can provide insight into the optimization of parameters, e.g. as discussed herein with the estimation of optimal combinations of wavelengths which is difficult or impossible using only empirical values.

The oxygenation can be calculated based on a collected number of measurements at a location of a selected blood vessel. In a preferred embodiment, the total integration time of measurements collected to determine the oxygenation in a selected blood vessel is dependent on a size of the selected blood vessel. Accordingly, the total integration time of measurements can be varied, and this affects the SNR of the image which can be used in the calculation of the uncertainty in oxygenation determination. For example, a size of the selected blood vessel can be determined, and the total integration time of measurements taken at the location of the blood vessel can be adjusted base on the determined size. The inventors find that the size of the blood vessel may affect an error in the calculated oxygenation using measurements of that blood vessel. This is explained e.g. by the analytical model described herein. By varying the number of measurements or integration time depending on the size of the blood vessel, an optimum can be found to achieve a minimum desired or acceptable error while avoiding unnecessary measurements. The inventors find that the error in the calculated oxygenation is higher when using the measurement values of a relatively small blood vessel. Accordingly, in some embodiments, the number of collected measurements or integration time for calculating the oxygenation is relatively high for a relatively small size of the selected blood vessel, and the number of collected measurements or integration time for calculating the oxygenation is relatively high for a relatively small size of the selected blood vessel.

In some preferred embodiments, the selection of wavelengths includes a first anisosbestic wavelength where oxyhaemoglobin has a lower absorption coefficient than deoxyhaemoglobin, and a second anisosbestic wavelength where oxyhaemoglobin has a higher absorption coefficient than deoxyhaemoglobin. By measuring both at a wavelength where the absorption coefficient increases with oxygenation and at another wavelength where the absorption coefficient decreases with oxygenation, the calculation may be improved over the whole range of possible oxygenations. For example, when calculating the ratio of intensities, even if the intensity for one or the numerator or denominator at one wavelength gets too low for a reliable measurement, the opposite will occur at the other wavelength.

In other or further embodiment, the selection of wavelengths may include at least one isosbestic wavelength where oxy- and deoxyhaemoglobin have the same absorption coefficient. Measurements at the isosbestic wavelength may be independent from the oxygenation, so only dependent on other parameters. This may help to isolate parameters and/or improve fitting constraints. In some embodiments, at least three different wavelengths are selected including at least one isosbestic wavelength, and at least two anisosbestic wavelengths preferably having opposite signs of relative absorption coefficients for oxyhaemoglobin compared to deoxyhaemoglobin. In this way, an oxygenation independent measurement at the isosbestic point may be further improved with the measurements at two anisosbestic points for covering the full range of oxygenations.

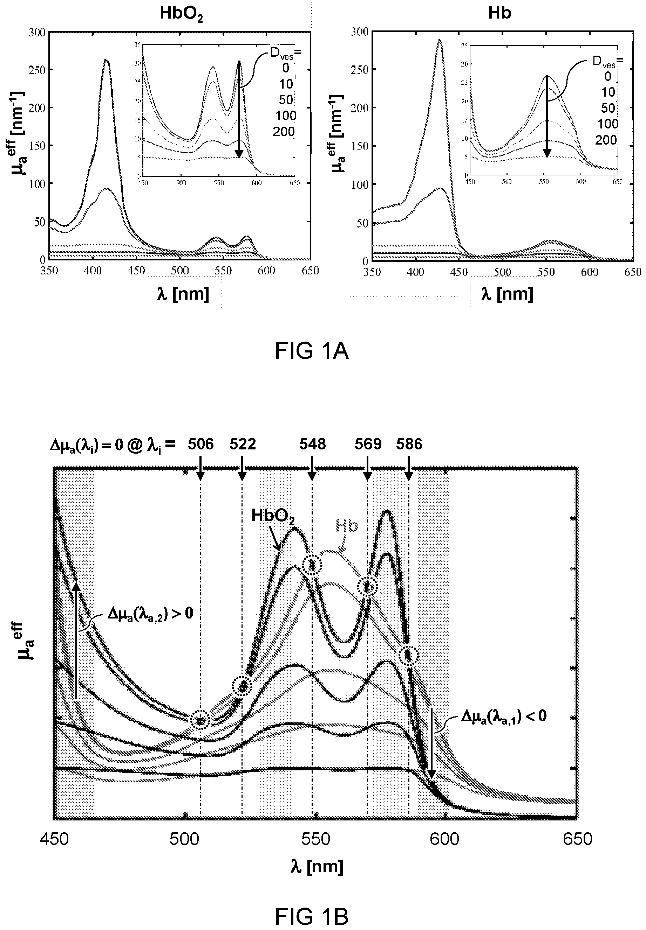

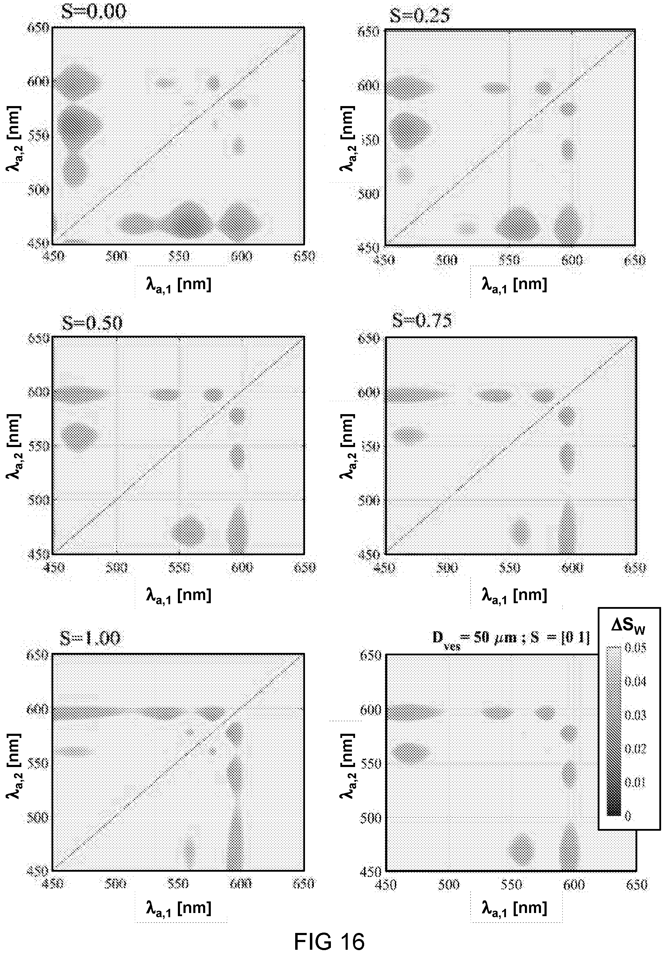

As will be further elucidated with reference to the detailed analysis below, an advantageous range for selecting an anisosbestic wavelength is preferably above 586 nm and more preferably around 596 nm. When using a second anisosbestic wavelength, this preferably this is selected below 506 nm, As shown e.g. in FIG 1B, the selection below the lowest isosbestic point and above the highest isosbestic point may provide relative large signals with opposite signs of the relative absorptions between the oxy- and deoxyhaemoglobin spectra. It is noted that the selection of discrete wavelengths relatively far apart is not obvious with regards to the generation thereof but provides particular advantages as described herein. This selection may also be combined with an isosbestic wavelength most preferably one of 506 nm, 522 nm, or 586 nm Alternatively, as illustrated e.g. in FIG 16, the second anisosbestic wavelength may also be selected e.g. in the ranges 522 - 548, preferably around 539 nm, or between 569 - 586, preferably around 578 nm.

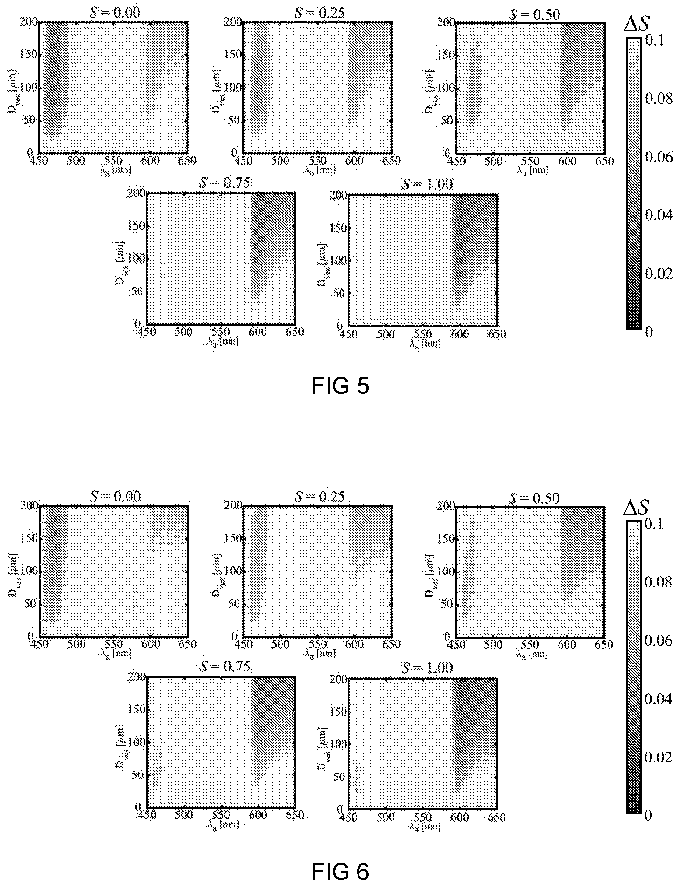

As discussed in further detail below the inventors find that for these particular selections the error in calculating the oxygenation is relatively low compared to other wavelengths. It will be appreciated that this particular preference of wavelengths is not obvious based on the difference of absorption spectra shown in FIG 1B, but is uniquely tied to the error analysis including the effects of relatively small vessel diameters, e.g. < 50μm. It will also be appreciated, e.g. from comparing different oxygenations in FIG 5 or FIG 16, that the optimal wavelength can be different for different oxygenations. Particularly note the position of the grey area which is predominant at wavelengths below 500 nm for low oxygenations but disappears for higher wavelengths and becomes predominant at wavelengths above about 590 nm. Also note that the optimal range particularly for the higher wavelengths can become rather narrow for lower vessel diameters.

In some embodiments, the analytical model includes an effective absorption coefficient of the blood vessel at the selection of wavelengths calculated as function of predetermined absorption coefficients of homogenously distributed oxy- and deoxyhaemoglobin at the selected wavelengths weighted according to the oxygenation defined as a fraction of one of the oxy- and deoxyhaemoglobin over a total haemoglobin. In other or further embodiments, the analytical model includes a correction factor depending on a size of the selected blood vessel to correct for a pigment packaging effect of absorbing haemoglobin molecules not being homogeneously distributed throughout the retinal tissue but contained in a discrete package of the selected blood vessel. Typically, the pigment packaging effect is more pronounced for a higher absorption and/or a larger size of the selected blood vessel. For example, the analytical model includes a term proportional with ( $1 - \exp( - D_{ves} \cdot \mu_{a,ves} )$ ), where $D_{ves}$ is proportional to a diameter the selected blood vessel, and $\mu_{a,ves}$ is proportional to an absorption coefficient of blood in the blood vessel according to a linear combination of oxy- and deoxyhaemoglobin spectra depending on the oxygenation. For example, this term appears in Equations 10-15 discussed below.

In some embodiments, the analytical model includes a relative optical density between an intensity measured at the blood vessel location compared to an intensity measured the tissue location for each wavelength. For example, the relative optical density is calculated as the logarithm of the ratio of tissue and blood vessel reflectivity at the same wavelength. In some embodiments, the intensity at the blood vessel location, for a particular wavelength is modelled as an intensity at the tissue location multiplied by a Lambert-Beer factor which is a function of an effective path length of the light contributing to the measured intensity and an average absorption coefficient along the path. In other or further embodiments, a dependence on the effective path length <L> is eliminated by assuming the scattering properties are the same for two or more wavelengths. In some embodiments, the analytical model includes calculating one or more ratios of optical density at two or more of the selected wavelengths.

In some embodiments, the analytical model includes calculating a statistical error in the calculated oxygenation. As explained with the examples below, the calculation of the statistical error may include the size of the selected blood vessel. In a preferred embodiment, the collecting of measurements may be continued until the calculated statistical error is below a predetermined threshold error, e.g. less than 10%, less than 5% or less than 1%.

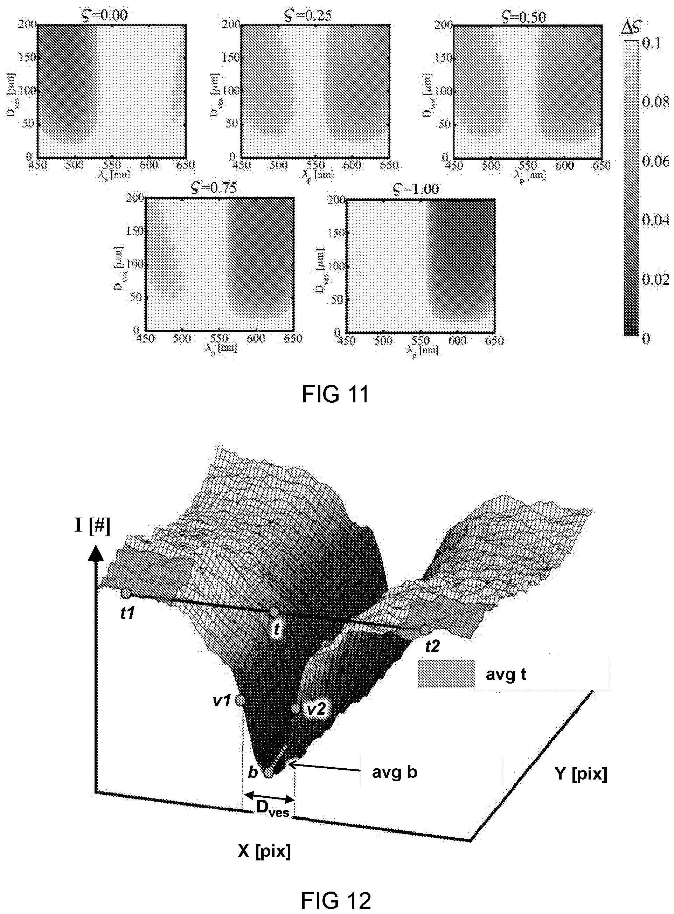

As described herein, calculation of the oxygenation takes into account the size of a selected blood vessel. The size can be separately determine for each vessel or even for different positions along a length of the same vessel (if the size is variable). In some embodiments, the size of the selected blood vessel is determined based on the position resolved measurements of the plurality of backscattered reflection intensities, e.g. as illustrated by reference to FIG 12. It will be appreciated that this is uniquely possible for the retina where the blood vessel can be distinguished. Alternatively, or additionally, the size of the selected blood vessel can be calculated or fitted based on the analytical model. For example, the fit can be overdetermined by using more wavelengths.

In accordance with these and other aspects the present disclosure may also provide a computer readable medium comprising software instructions, which when executed by a computer, cause the computer to perform the methods as described herein. The computer readable medium may also be integrated in corresponding system.

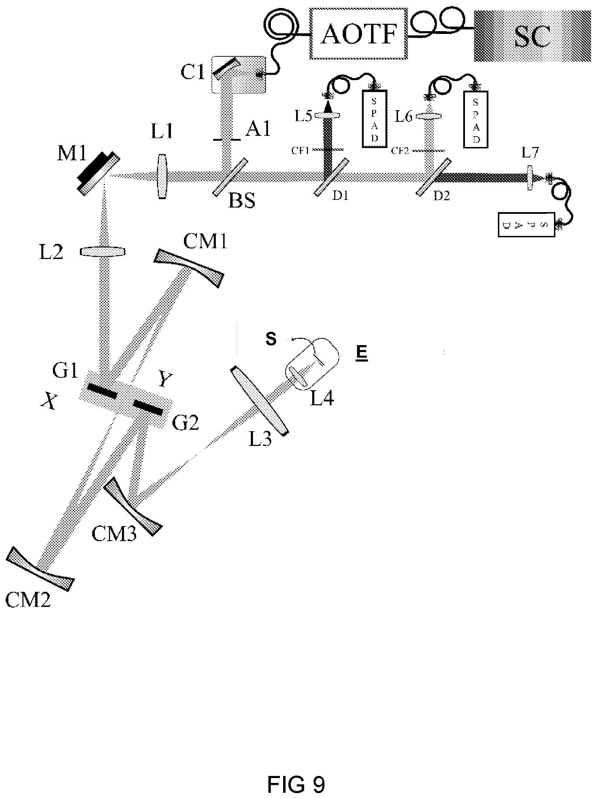

In accordance with these and other aspects the present disclosure may also provide a system for measuring retinal oxygenation. A light source can be configured to generate light essentially consisting of a selection of two or more discrete wavelengths. Projection optics can be configured to receive the light from the light source and illuminate a retinal tissue with blood vessels by the light. The projection can be configured to position-resolve at least a blood vessel location coinciding with a selected blood vessel and a tissue location coinciding with the retinal tissue without blood vessel. Collection optics can be configured to receive and measure backscattered reflection intensities of the light reflected of the retinal tissue with blood vessels, wherein the collection optics are configured to spectrally resolve the light for the selection of discrete wavelengths. A processor can be programmed to calculate the oxygenation based on the measured intensities, wherein the calculation includes an analytical model describing a dependence of the intensities on the oxygenation of blood in the selected blood vessel and a size of the selected blood vessel. Preferably, the system is configured for confocal measurement. More preferably, the system comprises a multi-wavelength Scanning Laser Ophthalmoscope e.g. as shown in FIG 9 or otherwise.

BRIEF DESCRIPTION OF DRAWINGS

These and other features, aspects, and advantages of the present disclosure will become better understood from the following description, appended claims, and accompanying drawing wherein:

FIG 1A illustrates the effect of pigment packing in retinal blood vessels. The figures show the effective absorption coefficient ($\mu_a^{eff}$) for a homogeneous distribution of blood (oxyhaemoglobin $HbO_2$, left; and deoxyhaemoglobin Hb, right) and the corresponding effective absorption coefficient of blood packed in discrete vessels of different vessel diameters $D_{ves}$ = 0 (homogeneous), 10,50,100,200 $\mu$m. The insets show a zoom in for 450-650 nm. It will be appreciated that there is a significant effect already of very small vessels e.g. at 10 $\mu$m especially at higher absorptions.

FIG 1B illustrates an overlay of the effective absorption spectra combining the insets of FIG 1A. This shows the isosbestic points (denoted by dotted circles at the isososbestic wavelengths $\lambda_i$ = 506 nm, 522 nm, 548 nm, 569 nm, and 586 nm) where the absorption depends on factors other than the 'oxygen saturation'. It will be appreciated that the pigment packaging does not affect the wavelength of the isososbestic points.

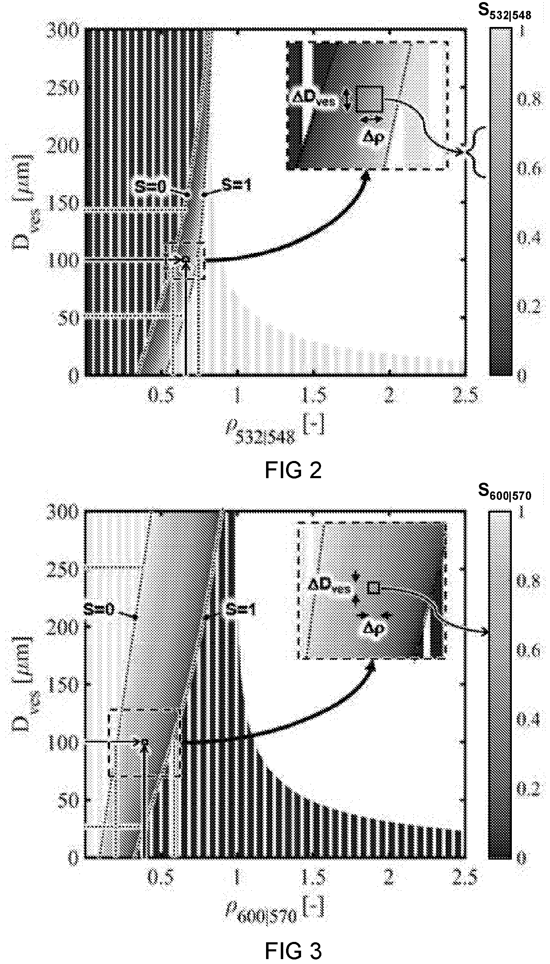

FIG 2 illustrates the mutual dependence of $D_{ves}$, $\rho_{532|548}$ and $S_{532|548}$ according to the model described herein by a plot of $S_{532|548}$ (gray scale) as a function of the blood vessel diameter $D_{ves}$ and $\rho_{532|548}$. The white space represents the region where there is no real-valued solution for the saturation. The real-valued solutions < 0 and > 1 were set to 0 and 1, respectively and are shown as striped regions. The narrow band of $\rho_{532|548}$ representing the whole range of saturation indicates that the estimation of $\rho_{532|548}$ needs to be extremely accurate for any reasonable estimation of saturation $S_{532|548}$. It also shows a strong dependence of the $S_{532|548}$ on $D_{ves}$.

FIG 3 illustrates the mutual dependence of $D_{ves}$, $\rho_{600|570}$ and $S_{600|570}$ according to the model described herein by a plot of $S_{600|570}$ (grayscale) as a function of the blood vessel diameter $D_{ves}$ and $\rho_{600|570}$. The white space represents the region where there is no real-valued solution for the saturation. The real-valued solutions < 0 and > 1 were set to 0 and 1 respectively and are shown as striped regions. It will be appreciated that, compared to FIG 2, the range of $\rho$ representing the saturation is nearly uniform across the different blood vessel sizes.

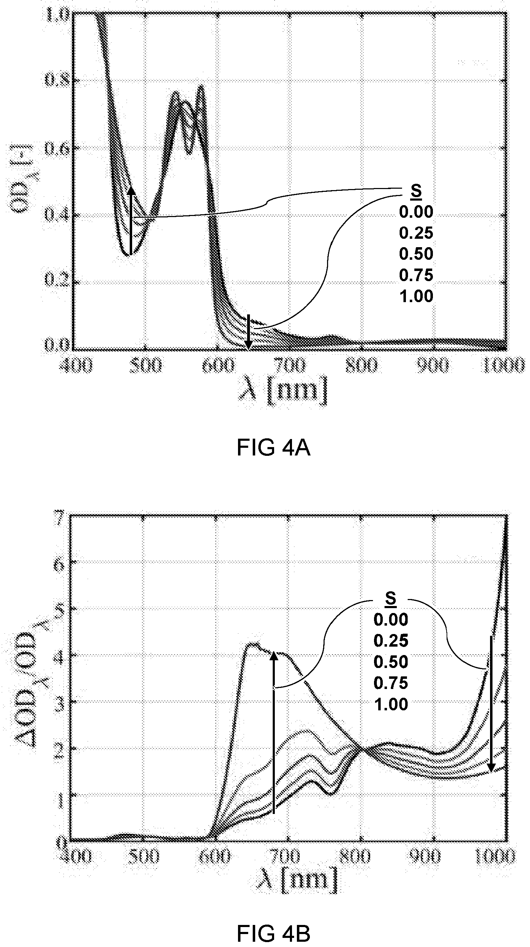

FIG 4A illustrates the OD's for different saturation values in a 50 $\mu$m diameter blood vessel plotted as a function of wavelength assuming $\langle L_\lambda \rangle$ = 300 $\mu$m (Eq .11). The effect of the correction factor applied to the absorption spectrum to include the pigment packaging effect is noticeable in the Soret band wavelengths around 430 nm. The ODs are insensitive to the change in saturation at these wavelengths due to very high absorption. The OD $\to$ 0 for wavelengths > 650 nm.

FIG 4B illustrates the relative error $\dfrac{\Delta OD_\lambda}{OD_\lambda}$ ($\Delta$OD calculated from Eq. 18 assuming $\dfrac{\Delta I\left(x_t, \lambda\right)}{I\left(x_t, \lambda\right)} = \dfrac{\Delta I\left(x_b, \lambda\right)}{I\left(x_b, \lambda\right)} = 0.01$ ) plotted for 400-1000 nm wavelength range increases rapidly for wavelengths above 600 nm because the OD approaches zero.

FIG 5 illustrates the saturation error ($\Delta$S) for different $\lambda_p$ when 506 nm is chosen as the isosbestic wavelength. The standard error on the mean recorded intensity values $(\dfrac{\Delta I}{I})$ was assumed to be 1%. All values of $\Delta S$ > 0.10 were made as 0.10.

FIG 6 illustrates saturation error ($\Delta$S) for different $\lambda_p$ when 548 nm is chosen as the isosbestic wavelength. The standard error on the mean recorded intensity values $(\dfrac{\Delta I}{I})$ was assumed to be 1%. All values of $\Delta S$ > 0.10 were made as 0.10.

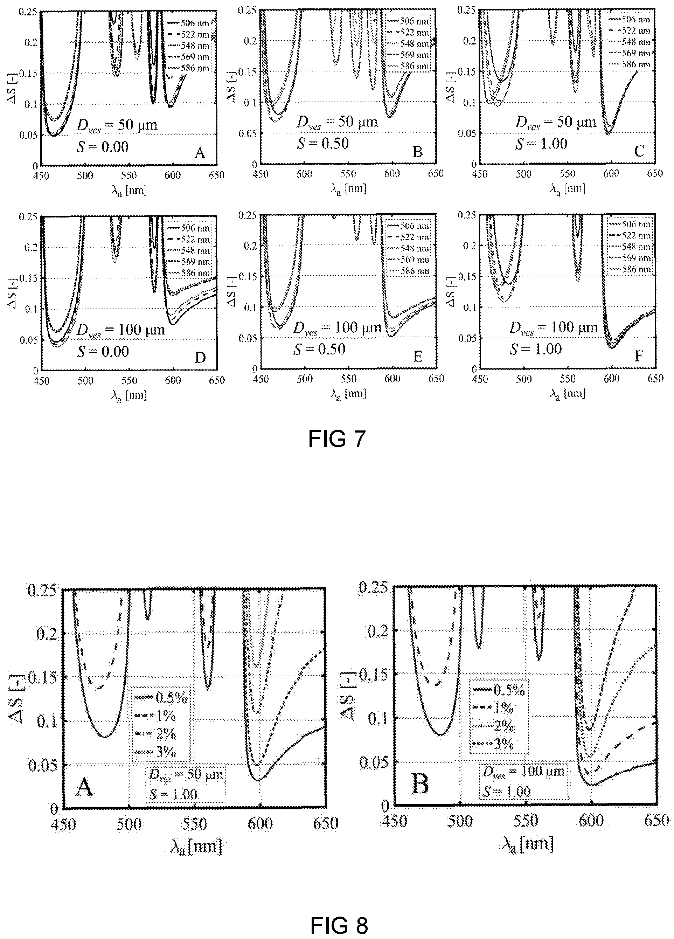

FIG 7 illustrates the saturation error ($\Delta$S) as a function of $\lambda_a$ for different saturation levels (A,D: S=0.00; B,E: S=0.50; C,F: S=1.00) for a 50 $\mu$m (A, B and C) blood vessel and a 100 $\mu$m (D, E and F) blood vessel when different isosbestic wavelengths in the 500 nm - 600 nm range were chosen as $\lambda_i$.

FIG 8 illustrates the saturation error ($\Delta S$) as a function of $\lambda_a$ when estimating full oxygenation (S = 1.00) for a 50 $\mu$m (A) and a 100 $\mu$m blood vessel (B) for different levels of % error in the intensities. The isosbestic wavelength chosen was 506 nm.

FIG 9 illustrates a schematic of a multiwavelength SLO which can be used as an embodiment for imaging: SC - Supercontinuum light source; AOTF-Acousto-optic tunable filter; L1 to L7 - Lenses; C1 - reflective collimator; M1-mirror; CM1 to CM3 - curved mirror; G1 and G2 - Galvo mirrors; A1- Aperture; DM1 and DM2 - Dichroic mirrors; CF1 and CF2 - additional spectral filters; SPAD- Single photon avalanche diode (detection unit), S=Sample, E=eye.

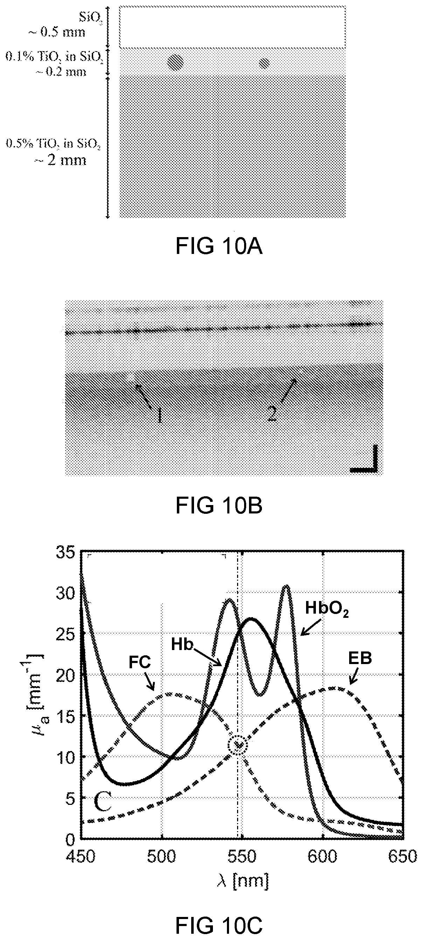

FIG 10A illustrates a layered structure of example phantoms: The phantoms were made to mimic the scattering properties of the retina and two cylindrical channels (circles) with a diameter of 90 $\mu$m and 140 $\mu$m were embedded in the scattering medium to mimic the blood vessels in the retina.

FIG 10B illustrates an OCT B-scan (in-house 1310 nm OCT, an average of 15 consecutive B scans) of the phantom with the 140 $\mu$m hollow (empty) channel (1) and 90 $\mu$m hollow (empty) channel (2). Scale bar is 300 $\mu$m in each direction

FIG 10C illustrates a comparison between absorption spectra of the dyes chosen for phantom measurements and blood

FIG 11 illustrates the saturation error ($\Delta S$) for different $\lambda_p$ when 548 nm is chosen as the isosbestic wavelength., $\Delta_\zeta$ > 0.10 were set to 0.10

FIG 12 illustrates a typical measurement of a (phantom) blood vessel intensity profile: The blood vessel absorption creates a dip in the intensity profile with a local minimum at point b. The OD is calculated as a ratio of the intensity of point $t$ (the linear approximation of the tissue intensity in the absence of the blood vessel) and point b. The points v1 and v2 are the points with a maximum slope along the vessel walls and $t1$ and $t2$ are the tissue locations used for the linear approximation of the tissue intensities.

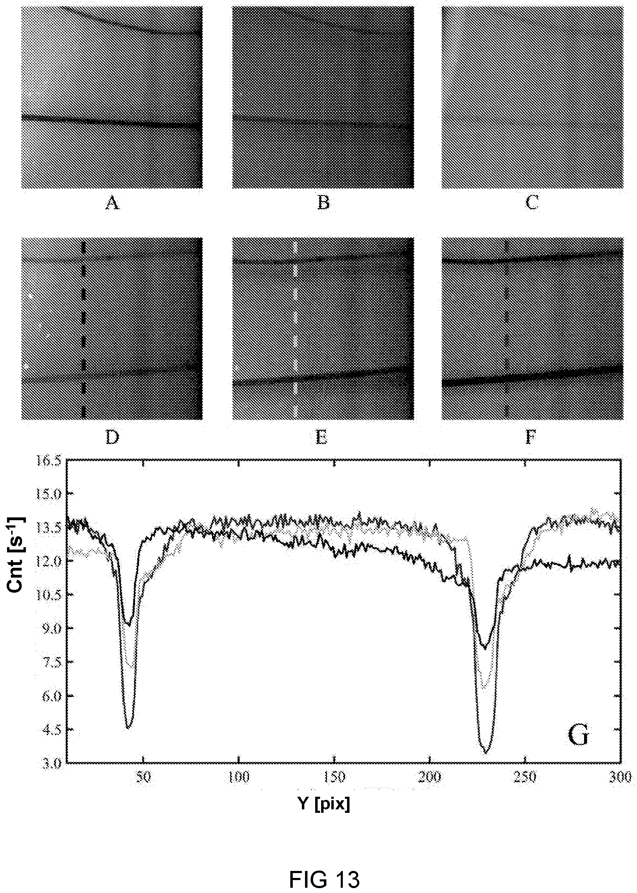

FIG 13 illustrates reflectance measurements of the phantoms in the model eye at three different wavelengths at 488 nm (A, D), 548 nm (B, E) and 612 nm (C, F). A - C shows the reflectance measurements for 100% food colouring ($\zeta$ = 1.00) showing high absorption at 488 nm (A). D - F shows the reflectance measurements for 0% food coloring ($\zeta$ = 0.00). The intensity profiles along the lines indicated in D - F is plotted in G - 488 nm (blue), 548 nm (green) and 612 nm (red).

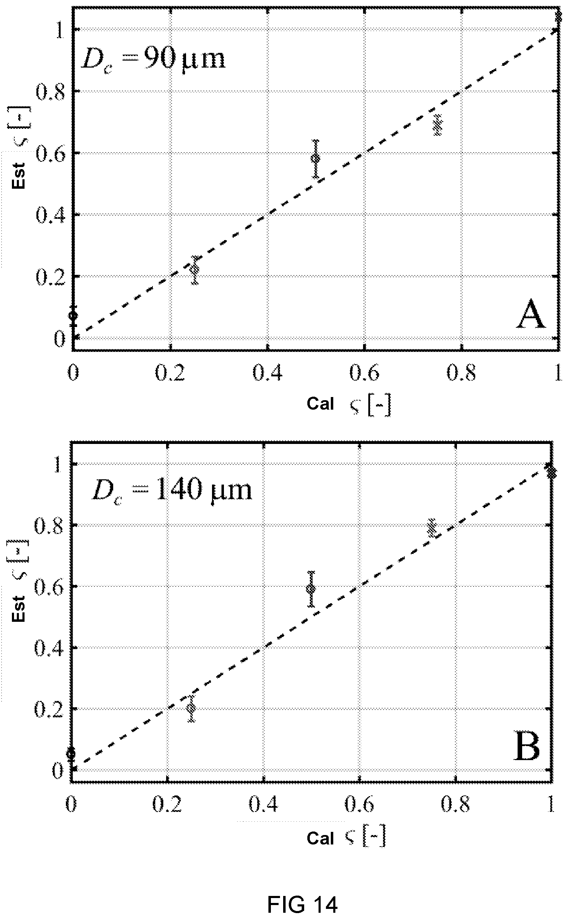

FIG 14 illustrates the estimated and measured saturation values for the dye combination in a 90 $\mu$m channel (top) and 140 $\mu$m channel (bottom) measured at 488 nm and 548 nm (o); 612 nm and 548 nm ($\times$).

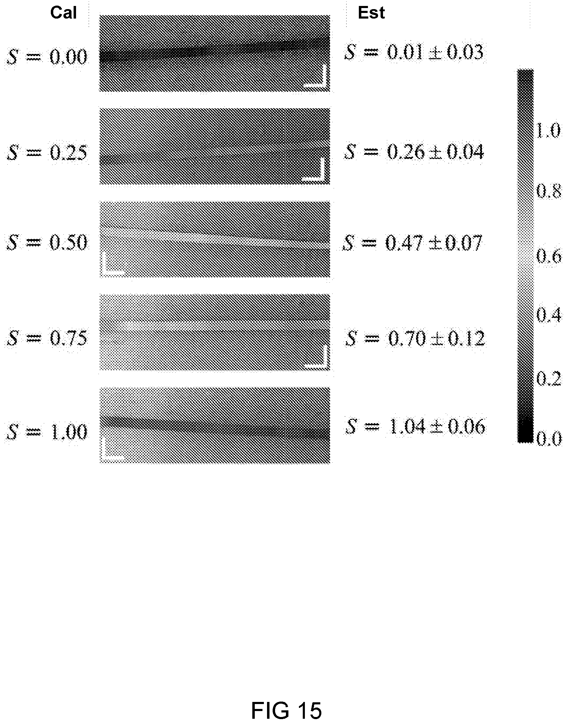

FIG 15 illustrates grayscale images of the saturation $\zeta$ superimposed on the reflectance image from the SLO.

FIG 16 illustrates graphs of saturation errors for different saturation values in a 50-micrometre blood using any two wavelengths between 450 and 650 nm. The greyscale bar represents values between 0 and 5 % error as indicated. On the bottom right, a weighted graph is illustrated over the whole range of saturation errors.

## DESCRIPTION OF EMBODIMENTS

One embodiment for measuring the absorbance of blood at a particular blood vessel in the retina may comprise measuring the intensity profile of the blood vessel as a function of the location on the retina. Without being bound by theory, it is generally found that the collected intensity $I(x_o,\lambda)$ recorded at a spatial location $x_o$ in the retina for a given wavelength $\lambda$ of illuminated light may depend on the incident illumination intensity $I_o(x_i,\lambda)$ at the point $x_i$ and the back-scattered reflectance function of the retina, denoted herein as $R(x_o, x_i, \lambda)$. In some embodiments, the retina is illuminated by a narrow, focused beam of light incident on the retina at $x_i$ and the detection pinhole collects from a larger volume around $x_o$ compared to the illumination area.

For example, let $x_o$ be the averaged volume of tissue surrounding $x_o$ that contributes to the collected intensity $I(x_o, \lambda)$. The extent of volume averaging for any location with respect to the spatial distribution of the optical properties at that location may be determined by the incident illumination profile $I_o(x_i, \lambda)$ and the detection pinhole. Under such conditions, the collected intensity I can e.g. be modeled as,

**Equation 1**

$$I(x_o,\lambda) = \int I_o(x_i,\lambda) \cdot R(x_i, x_o, \lambda) \cdot dx_i = I_o(x_o,\lambda) \cdot R(\overline{x_o},\lambda)$$

where the function $R\left(\overline{x_o},\lambda\right)$ describes the reflection averaged over a small tissue volume at the location $x_o$ due to scattering and absorption.

Preferred embodiments as described herein may involve comparing measurements a different locations, in particular comparing one measurement at a location coinciding with a blood vessel with another measurement at another location of adjacent tissue, without blood vessel. For example, let $x_b$ and $x_t$ denote the recorded locations at the centre of a blood vessel and in the adjacent tissue, respectively.

In some embodiments, the light collected from a tissue location $x_t$ can be modeled as

**Equation 2**

$$I\left(x_t,\lambda\right) \propto I_o\left(x_t,\lambda\right)R\left(\overline{x_t},\lambda\right) = I_o\left(x_t,\lambda\right)R\left(\left(\mu_s(\overline{x_t},\lambda)\cdot p(\overline{x_t},\theta,\lambda)\right)\right)$$

where in this case $R(\overline{x_t}, \lambda)$ is written as a function of the average scattering coefficient $\mu_s(\overline{x_t},\lambda)$ of the volume of tissue around $x_t$ (denoted by $\overline{x_t}$) at the wavelength $\lambda$ and of the scattering phase function ($p(\overline{x},\theta,\lambda)$) of the same tissue volume. In this case it is assumed that there is negligible or no localized absorber (haemoglobin) in the tissue volume.

In other or further embodiments, the light collected from the centre of a blood vessel location $x_b$ can be modelled as

**Equation 3**

$$I\left(x_b,\lambda\right) \propto I_o\left(x_b,\lambda\right)\cdot R\left(\overline{x_b},\lambda\right) = I_o\left(x_b,\lambda\right)\cdot R\left(\mu_s(\overline{x_b},\lambda), p(\overline{x_b},\theta,\lambda), \mu_a(\overline{x_b},\lambda)\right)$$

where in this case $R(\overline{x_b}, \lambda)$ is written as a function of the average scattering coefficient $\mu_s(\overline{x_b},\lambda)$ of the volume of tissue around $\overline{x_b}$ (denoted by $\overline{x_b}$ ) at the wavelength $\lambda$, of the scattering phase function $p(\overline{x_b},\theta,\lambda)$ of the same tissue volume and of the effective absorption coefficient of the tissue volume containing the blood vessel $\mu_a(\overline{x_b},\lambda)$.

In some embodiments, the function $R(\mu_s(\overline{x_b},\lambda),p(\overline{x_b},\theta,\lambda),\mu_a(\overline{x_b},\lambda))$ can be modeled using the *Beer-Lambert's law,* e.g. exponentially decreasing as a function of the average or effective absorption coefficient and the effective path. For example, the following equation can be used

**Equation 4**

$$R\left(\mu_s(\overline{x_b},\lambda), p(\overline{x_b},\theta,\lambda), \mu_a(\overline{x_b},\lambda)\right) =$$

$$R\left(\left(\mu_s(\overline{x_b},\lambda)\cdot p(\overline{x_b},\theta,\lambda)\right)\right)\times\exp\left(-\langle L\rangle\cdot\mu_a(\overline{x_b},\lambda)\right)$$

where in this case the absorption of the blood within the blood vessel volume is modeled denoting $\langle L\rangle$ as the effective path length of the photons travelling through the volume $\overline{x_b}$ , and $\mu_a(\overline{x_b},\lambda)$ the effective absorption coefficient of that volume.

In some embodiments, the retinal scattering properties can be approximated or modeled assuming that they are virtually identical except for the presence of a blood vessel. In such embodiments, the reflected intensity at a blood vessel can be expressed as the reflectivity of the retinal tissue multiplied with an absorption factor,

**Equation 5**

$$I(x_b,\lambda) = I(x_t,\lambda)\cdot\exp\left(-\langle L\rangle\cdot\mu_a(\overline{x_b},\lambda)\right)$$

where it is assumed all additional reflections occurring internally in the system due to optics and the stray reflections from the cornea are accounted for, e.g. by subtracting a reference measurement.

As described herein, the absorption coefficient $\mu_a(\overline{x_b},\lambda)$ of the blood is considered a function of the saturation (S) of the blood (i.e., the fraction of the oxygenated haemoglobin concentration to the total concentration of haemoglobin) and can be modeled in some embodimetns as a linear combination of the oxygenated and deoxygenated spectra. For example, the following model can be used

**Equation 6**

$$\mu_a(\overline{x}_b, \lambda) = v\left(S \cdot \mu_a^{HbO_2}(\lambda) + (1-S) \cdot \mu_a^{Hb}(\lambda)\right)$$

where $V$ is the fraction of the blood volume in the total volume considered. For example, in some embodiments the parameter V can be approximated as being proportional to the ratio of the vessel diameter and the effective path length, $D_{ves}/\langle L \rangle$. The parameters $\mu_a^{HbO_2}(\lambda)$ and $\mu_a^{Hb}(\lambda)$ are the absorption coefficients of oxy- and deoxyhaemoglobin, respectively (e.g. assuming 150 mg of haemoglobin in 1 mL of blood) and are well-known functions of wavelength. Optionally, additional spectra may be included to further improve the model although possibly at the cost of additional parameters.

In some embodiments, it is found advantageous to use the relative optical density $OD_\lambda$ of the tissue at the blood vessel location compared to the surrounding tissue at a particular wavelength. For example, this can be expressed as the logarithm of the ratio of tissue and blood vessel reflectivity at the same wavelength:

**Equation 7**

$$OD_\lambda = \ln\left(\frac{I(x_t, \lambda)}{I(x_b, \lambda)}\right) = \langle L_\lambda \rangle \cdot \mu_a(\overline{x}_b, \lambda)$$

In some embodiments, Eqs. 6 and 7 can be combined to yield

**Equation 8**

$$OD_\lambda = \langle L_\lambda \rangle \cdot v \cdot \left(S \cdot \mu_a^{HbO_2}(\lambda) + (1-S) \cdot \mu_a^{Hb}(\lambda)\right)$$

Equation 8 represents an oximetry equation where $I(x_t, \lambda)$ and $I(x_b, \lambda)$ are e.g. obtained from the retinal image to estimate the $OD_\lambda$. Equation 8 still assumes a homogenous distribution of haemoglobin throughout the probed tissue volume containing the blood vessel. However, the inventors find that the absorbing haemoglobin molecules are not homogeneously distributed throughout the tissue volume containing the blood vessel but are contained in a discrete package (the blood vessel). This packaging of absorbing molecules is found to flatten the absorption spectrum particularly for high absorption coefficients by reducing the effective light absorption, and is referred herein as the pigment packaging effect'.

In some embodiments, the pigment packaging effect can be modelled e.g. through the incorporation of a correction factor to the absorption spectrum. For example, the absorption coefficient in Eq. (6) can be modeled as

**Equation 9**

$$\mu_a(\overline{x}_b, \lambda) = v\left(S \cdot \mu_a^{HbO_2}(\lambda) + (1-S) \cdot \mu_a^{Hb}(\lambda)\right) \cdot C_{corr}(S, \lambda)$$

In a preferred embodiment, the correction is described by an anytical function or model dependent on the vessel diameter $D_{ves}$. For example, the following approximation -as described for other applications by Rajaram, et al. [Lasers Surg. Med. 42, 680-688 (2010)]- can be used

**Equation 10**

$$C_{corr}(S, \lambda) = \frac{1 - \exp\left(-D_{ves} \cdot \left(S \cdot \mu_a^{HbO_2}(\lambda) + (1-S) \cdot \mu_a^{Hb}(\lambda)\right)\right)}{D_{ves} \cdot \left(S \cdot \mu_a^{HbO_2}(\lambda) + (1-S) \cdot \mu_a^{Hb}(\lambda)\right)}$$

where $D_{ves}$ is the blood vessel diameter. Of course, also another or further identifiable geometry e.g. size of the vessel may be incorporated in the model. It will be particularly appreciated that blood vessels may be easily recognized at the surface of retinal tissue which is rather unique. This makes the field of retinal oximetry uniquely suitable for the application of models incorporating the effects of vessel geometry on the effective absorption , as the inventors realized.

In some embodiments, Eqs. (7), (9) and (10), can be combined to yield an improved model for the measured relative optical density $OD_\lambda$ at a particular wavelength $\lambda$ including the pigment packaging effect of a selected vessel with diameter $D_{ves}$:

**Equation 11**

$$OD_\lambda = \frac{\langle L_\lambda \rangle \cdot v}{D_{ves}} \cdot \left(1 - \exp\left(-D_{ves} \cdot \left(S \cdot \mu_a^{HbO_2}(\lambda) + (1-S) \cdot \mu_a^{Hb}(\lambda)\right)\right)\right)$$

It will be appreciated these or other models including the vessel diameter as a parameter for correcting the modeled absorption for each selected vessel may improve accuracy over conventional models. For example, such models can be used to fit multivariate measurement data using the saturation S as fit parameter. For example, the data include may measured optical densities at various locations and wavelengths, e.g. at or adjacent one or more selected vessels with known, modeled, or measured diameters. In some embodiments, the vessel diameter can even be another fit parameter or eliminated as parameter by combining equations for multiple, e.g. three or more wavelengths..

In some embodiments, the model can be further refined, e.g. eliminating parameters, by defining the ratio $\rho_{\lambda 1}|_{\lambda 2}$ of ODs at two wavelengths $\lambda_1$ and $\lambda_2$ as

**Equation 12**

$$\rho_{\lambda_1 | \lambda_2} = \frac{OD_{\lambda 1}}{OD_{\lambda 2}} = \frac{\langle L_{\lambda_1} \rangle \cdot \left(1 - \exp\left(-D_{ves} \cdot \left(S \cdot \mu_a^{HbO_2}(\lambda_1) + (1-S) \cdot \mu_a^{Hb}(\lambda_1)\right)\right)\right)}{\langle L_{\lambda_2} \rangle \cdot \left(1 - \exp\left(-D_{ves} \cdot \left(S \cdot \mu_a^{HbO_2}(\lambda_2) + (1-S) \cdot \mu_a^{Hb}(\lambda_2)\right)\right)\right)}$$

Although not mathematically necessary, it is preferred for some embodiments to choose an 'isosbestic' point as one of the wavelengths for imaging to eliminate the saturation dependence on one of the ODs. It will be appreciated that at an isosbestic wavelength $\lambda_i$, $\mu_a^{HbO_2} = \mu_a^{Hb} = \mu_a^i$ and hence,

**Equation 13**

$$OD_{\lambda_i} = \frac{\langle L_{\lambda_i} \rangle \cdot v}{D_{ves}} \cdot \left(1 - \exp\left(-D_{ves} \cdot \mu_a^i(\lambda_i)\right)\right)$$

This can simplify the expression of at one of

For example, the ratio of ODs at two wavelengths where one of the wavelengths is isosbestic can be modelled as,

**Equation 14**

$$\rho_{\lambda_a | \lambda_i} = \frac{OD_{\lambda_a}}{OD_{\lambda_i}} = \frac{\langle L_{\lambda_p} \rangle \cdot \left(1 - \exp\left(-D_{ves} \cdot \left(S \cdot \mu_a^{HbO_2}(\lambda_a) + (1-S) \cdot \mu_a^{Hb}(\lambda_a)\right)\right)\right)}{\langle L_{\lambda_i} \rangle \cdot \left(1 - \exp\left(-D_{ves} \cdot \mu_a^i(\lambda_i)\right)\right)}$$

where $\lambda_a$ denotes an oxygen saturation sensitive 'anisosbestic' wavelength.

In some embodiments, these two wavelengths are selected close enough such that the scattering properties can be assumed to be the same, i.e., $\langle L_{\lambda_a} \rangle$; $\langle L_{\lambda_i} \rangle$. Expressing S as a function of $\rho$ in Equation 14 we get,

**Equation 15**

$$S_{\lambda_a | \lambda_i} = \frac{D_{ves} \cdot \mu_a^{Hb}(\lambda_a) + \ln\left(1 + \rho_{\lambda_a | \lambda_i} \cdot \exp\left(-D_{ves} \cdot \mu_a^i(\lambda_i)\right) - \rho_{\lambda_a | \lambda_i}\right)}{D_{ves} \cdot \left(\mu_a^{Hb}(\lambda_a) - \mu_a^{HbO_2}(\lambda_a)\right)}$$

FIG 2 shows a plot of the variation of $S_{532|548}$ (grayscale) as a function of the parameters $\rho_{532|548}$ and $D_{ves}$. The white space in the graph represents the region where a real-valued solution for $S_{532|548}$, is not possible for the particular combination of $\rho_{532|548}$ and $D_{ves}$. For simplicity, the real-valued solutions S < 0 and S > 1 (shaded regions in) were set to0 and 1, respectively, as it is physically impossible to have these saturation values. FIG 2 shows that a narrow band of $\rho$ values represents the whole range of saturation values [0 1], particularly for large vessel diameters. This indicates that the an extremely accurate measurement of $\rho_{532|548}$ is required to estimate the correct saturation value for this wavelength combination. Moreover, for a given rho, the saturation strongly depends on the blood vessel diameter.

FIG 3 shows a plot of $S_{600|570}$ as a function of $\rho_{600|570}$ and $D_{ves}$, showing a nearly uniform, broad range of $\rho_{600|570}$ values for variations in $S_{600|570}$ spanning the [0 1] range, for a large range of blood vessel diameters.

It will be appreciated that if the bands of $\rho$ values corresponding to the same saturation S are vertically straight, the saturation estimation may be relatively insensitive to the blood vessel diameter as a wide range of blood vessels will yield the same $S$ for a given $\rho$. So FIGs 2 and 3 can give an overall awareness in choosing the right wavelengths.

The selection of wavelengths can be further improved e.g. using a detailed error analysis of Eq. 15. For example, the error in saturation $\Delta S$ can be describe by the error propagation formula as

**Equation 16**

$$|\Delta S| = \sqrt{\left(\frac{\partial S}{\partial \rho}\right)^2 \cdot (\Delta \rho)^2 + \left(\frac{\partial S}{\partial D_{ves}}\right)^2 \cdot (\Delta D_{ves})^2}$$

It will be noted that here the error $\Delta S$ can be function of $S$, $D_{ves}$ and $\lambda_a$ for a chosen $\lambda_i$. Now we proceed to analyse each term in Equation 16 and its contribution to the saturation error $\Delta S$.

In some embodiments, e.g. an image based estimation of the blood vessel diameter $D_{ves}$ using an SLO technique, a constant error on the estimation of the diameter ($\Delta D_{ves}$) can be assumed e.g. 12 $\mu$m. The error on $\rho_{\lambda_a|\lambda_i}$ is then given by error propagation as,

**Equation 17**

$$\Delta \rho_{\lambda_a|\lambda_i} = \frac{OD_a}{OD_i} \cdot \sqrt{\left(\frac{\Delta OD_a}{OD_a}\right)^2 + \left(\frac{\Delta OD_i}{OD_i}\right)^2}$$

and the error in the ODs is given by the underlying error of the reflectivities $I(x_t,\lambda)$ and $I(x_b,\lambda)$ as

**Equation 18**

$$\Delta OD_\lambda = \sqrt{\left(\frac{\Delta I(x_t,\lambda)}{I(x_t,\lambda)}\right)^2 + \left(\frac{\Delta I(x_b,\lambda)}{I(x_b,\lambda)}\right)^2}$$

From Eq. 17 we see that $\Delta \rho \to \infty$ when OD $\to$ 0 because of the 1/(OD) factor.

FIG 5 shows the OD for a 50 $\mu$m blood vessel for different saturations and wavelengths assuming $\langle L \rangle$ = 0.3 mm and a fraction of blood in the total tissue volume probed, v = 0.17 [-] (50$\mu$m/300$\mu$m) . Due to very low absorption at wavelengths > 650 nm, there is a poor contrast between the recorded intensity at the tissue location and the blood vessel location which results in OD $\to$ 0. This results in large errors in $\rho$ and therefore, in the saturation estimation.

Although FIG 4B shows the relative error on OD is small for wavelengths < 450 nm, there are several reasons to also avoid wavelengths <450 nm for oximetry. Absorption is very high in the Soret band (430 nm) resulting in most of the light being absorbed by the blood resulting in a very poor signal to noise ratio at the blood vessel locations. The haemoglobin pigment packing makes the difference in OD due to the change in saturation indistinguishable. Most importantly, the dominant blue light hazard at the wavelengths < 450nm substantially limits the maximum permissible exposure of light for these wavelengths. Thus, in some embodiments it is preferred to exclude wavelengths < 450 nm and > 650 nm. From Eqs. 17 and 18, it is evident that the standard error of the mean recorded intensities in the detector

$$\left(\frac{\Delta I}{I} \text{ or } \frac{\sigma_I}{\langle I \rangle}\right)$$ propagates to an error in the OD's and then to an error in $\rho$, and thus contributes to the saturation error $\Delta S$.

For example, a 1.0% error in the intensities $\left(\frac{\Delta I}{I} = 0.01\right)$ propagates to an error in the ODs ($\Delta OD_\lambda = 0.014$).

If $OD_{\lambda_a} = 0.5$ and $OD_{\lambda_i} = 0.7$, this gives 3.4% error in $\rho\left(\frac{\Delta\rho}{\rho} = 0.034\right)$ according to Eq. 18, This error in $\rho$ propagates to saturation error $\Delta S$ in a complex manner through Equation 12.

FIGs 5 and 6 show grayscale graphs of the calculated errors in saturation as a function of $D_{ves}$ and $\lambda_a$ considering a 1% error ($\sigma_I / \langle I \rangle$) in the intensities when 506 nm (lowest $\mu_a^i$ in the 450 nm - 650 nm interval) and 548 nm (highest $\mu_a^i$ in the 450 nm - 650 nm interval) were used as isosbestic points, respectively. For simplicity, all the saturation error values ($\Delta S$) > 0.10 were set to 0.10 to emphasize the combinations with accuracy better than 0.1. From Figure 5, we see that when 506 nm is used as an isosbestic wavelength, there are two spectral bands optimal for oximetry. For low saturation values (0.00 - 0.25), either of the bands, 460 nm - 480 nm or 594 nm -600 nm could be used. But for higher saturation levels (0.75 - 1.00), only the 594 - 600 nm gives $\Delta S$ < 0.1.

When 548 nm is used as an isosbestic wavelength (FIG 6), 594 - 600 nm may not be ideal for detecting the low saturation values for blood vessels with a diameter less than 150 $\mu$m.

FIG 7 shows the saturation error for a 50 $\mu$m (Fig.7A-7C) and a 100 $\mu$m (Fig.7D-7F) blood vessel for different saturation levels S and using different isosbestic wavelengths. From these graphs, it may be noted that all five isosbestic wavelengths under consideration may perform similarly for estimating fully oxygenated blood (S = 1.00). However, for estimating low saturation values, 522 nm, 586 nm and 506 nm perform marginally better than 548 nm and 569 nm. One embodiment may thus include selecting at least one isosbestic point at a wavelength $\lambda_i$ = 522 nm, 586 nm or 506 nm.

Since the standard error of the mean intensities can have a significant bearing on the saturating error, a large number of points may need to be averaged both at the blood vessel locations and the tissue locations. This can also be achieved by averaging a large number of images and thereby minimizing the error of the mean intensity to acceptable levels.

FIG 8 shows $\Delta S$ values as a function of $\lambda_a$ ($\lambda_i$ = 506 nm) for a fully oxygenated 50 $\mu$m and 100 $\mu$m blood vessel for different levels of the standard error of the mean. It can be seen in this figure, that in order to reach saturation accuracy levels better than 5% for 100% oxygenation when measuring near 594 nm, the required accuracy level of the mean intensity was 1 % for 100 $\mu$m and even for 50 $\mu$m vessels.

It will be appreciated that the analysis as described for one isosbestic and one anisosbestic wavelength may apply mutatis mutandis to another embodiment where two anisosbestic wavelengths are used, e.g. as discussed in the following.

For example, let the optical density at any measurement wavelength $\lambda$ be given by the log of the ratio of intensity at tissue location to blood location.

**Equation 19**

$$OD_\lambda = \ln\left(\frac{I(x_t, \lambda)}{I(x_b, \lambda)}\right)$$

Use the parameter $\rho$ as the ratio of two ODs at two different wavelengths $\lambda_1$ and $\lambda_2$.

**Equation 20**

$$\rho = \frac{\left[OD_{\lambda_1}\right]}{\left[OD_{\lambda_2}\right]};$$

where

### Equation 21

$$\rho(\lambda_1, \lambda_2, S, D_{ves}) = \frac{\left(1 - e^{-D_{ves} \cdot (S \cdot \mu_a^{HbO_2}(\lambda_1) + (1-S) \cdot \mu_a^{Hb}(\lambda_1))}\right)}{\left(1 - e^{-D_{ves} \cdot (S \cdot \mu_a^{HbO_2}(\lambda_2) + (1-S) \cdot \mu_a^{Hb}(\lambda_2))}\right)}$$

The oxygen saturation S can be found from this equation. For example, the error in $\rho$ can be written as,

### Equation 22

$$\Delta\rho = \sqrt{\left(\frac{\partial\rho}{\partial S}\right)^2 \cdot (\Delta S)^2 + \left(\frac{\partial\rho}{\partial D_{ves}}\right)^2 \cdot (\Delta D_{ves})^2}$$

So, the saturation error can be expressed by rearranging the equation as,

### Equation 23

$$\Delta S = \pm \frac{\sqrt{(\Delta\rho)^2 - \left(\frac{\partial\rho}{\partial D_{ves}}\right)^2 \cdot (\Delta D_{ves})^2}}{\left(\frac{\partial\rho}{\partial S}\right)}$$

FIG 16 illustrates plots of the saturation error for a preferred embodiment with typical values a 50 $\mu$m blood vessel, $\Delta D_{ves}$ = 12 $\mu$m and SNR = 20 dB. The darker spots in the graphs indicate advantageous combination of wavelengths where the saturation error is lower than five percent for each of the indicted saturation values. The graphs is found to be representative of the relatively smaller blood vessels, e.g. <50 $\mu$m, where the oxygenation measurement is most important.

It will be noted that the positions of the optimal wavelengths may vary for different saturations. To select optimum values for the whole range, we can e.g. weight each of the graphs above to combine them into a single graph to make it easier to select wavelengths for a particular $D_{ves}$.

### Equation 24

$$\Delta S_w = \frac{w_0 \cdot \Delta S_0 + w_{25} \cdot \Delta S_{25} + w_{50} \cdot \Delta S_{50} + w_{75} \cdot \Delta S_{75} + w_{100} \cdot \Delta S_{100}}{w_0 + w_{25} + w_{50} + w_{75} + w_{100}}$$

where $\Delta S_0$, $\Delta S_{25}$, $\Delta S_{50}$, $\Delta S_{75}$ and $\Delta S_{100}$ are the saturation errors at 0%, 25%, 50%, 75% and 100 % saturation levels and w's are the corresponding weights. One example of a weighted graph is shown in FIG 16 bottom right. Here we used weights $w_0$=0.4; $w_{25}$ = 0.6; $w_{50}$ = 0.8; $w_{75}$ =1.0 and $w_{100}$ = 1.0 where weights favor wavelengths more accurate for higher saturation values

In summary, we have considered the case where the oximetry is performed at any two wavelengths in particularly focusing on the advantageous range between 450 and 650 nm. First, we considered the case where at least one of the wavelengths was an isosbestic wavelength. Then we considered where both wavelengths were anisosbestic.

According to the model calculations as e.g. illustrated by FIGs 2-8,16, this may lead to the following table summarizing optimal wavelength combinations for minimal error, e.g. $\Delta S$<5%:

Table 1. Optimal wavelength values and ranges for vessel <50$\mu$m

| | first anisosbestic wavelength ($\lambda_{a,1}$) | second anisosbestic wavelength ($\lambda_{a,2}$) | isosbestic wavelength ($\lambda_i$) |
|---|---|---|---|
| one isosbestic and one anisosbestic wavelength | 596 $\pm$ 7 nm | - | 506 nm |
| | | | 522 nm |
| | | | 548 nm |
| | | | 569 nm |
| | | | 586 nm |
| one isosbestic and two anisosbestic wavelengths | 596 $\pm$ 7 nm | 466 $\pm$ 16 nm | 506 nm |
| | | | 522 nm |
| | | | 586 nm |
| two anisosbestic wavelengths | 596 $\pm$ 7 nm | 466 $\pm$ 16 nm | - |
| | | 539 $\pm$ 8 nm | |
| | | 578 $\pm$ 7 nm | |

It will be appreciated that the $\Delta$S threshold may be related to the SNR and the vessel diameter. The wavelength bands detailed in the table represent the wavelengths that correspond to the smallest SNR and therefore the lowest integration time or the lowest amount of photon exposure to the retina to achieve any chosen $\Delta$S threshold. Of course, if a higher error $\Delta$S is acceptable, the plus-minus ($\pm$) ranges as indicated above may be higher, e.g. $\pm$10nm or $\pm$15nm instead of $\pm$ 7nm or $\pm$ 7nm, e.g. $\pm$20 nm or $\pm$25 nm instead of $\pm$16 nm. It is noted there also appears in FIG 16 a an optimum for two anisosbestic wavelengths of 466 $\pm$ 16 nm and 560 $\pm$ 10 nm but this may have relatively poor signal at higher saturation e.g. S=1.00 so is less preferred than the indicated values.

While selecting wavelengths outside optimum wavelength bands may sometimes also allow achieving a desired $\Delta$S threshold, this is typically at the expense of higher exposures or longer integration times in order to achieve a higher SNR for the same $\Delta$S threshold compared to our selected optimum wavelength bands. The combinations of wavelength bands shown in the graphs and table thus represent a notable progress resulting from the earlier presented careful analytical derivation of the retinal oximetry algorithm by including the pigment packaging factor. Further, it will be appreciated that even if similar wavelength combinations were previously used by chance, they could still report incorrect saturation values if they did not use the appropriate retinal oximetry algorithm that includes the pigment packaging factor.

In the following, an example system is described for implementing the methods as described herein on real or model samples. While this is one of the preferred embodiments, it will be appreciated however that various other systems can be envisaged to provide similar measurements. In a preferred embodiment, a multi-wavelength Scanning Laser Ophthalmoscope (SLO) is used. In some embodiments, the system may continuously acquire of en *face* images of the retina. Optionally, by implementing confocal pinholes, high contrast images can be obtained and the system is generally referred to as a confocal SLO (cSLO). Compared to a fundus camera, an SLO may have a superior resolution due to scanning and confocal collection. A typical cSLO uses a single wavelength illumination to produce high-quality en *face* images of the retina. Acquiring spectral information at each spatial location on the retina expands the diagnostic capabilities of a cSLO and consequently, it can be adapted to accommodate multiple wavelengths by multiplexing temporally or spectrally.

One embodiment of an cSLO is schematically illustrated in FIG. 9. While this is a preferred embodiment, it will be appreciated that various of the components shown can be omitted or replaced with equivalent components depending on the applications. In the embodiment shown, the system comprises a supercontinuum (SC) laser source as the light source. In the embodiment shown, the system comprises an acoustic-optic tunable filter, (AOTF) coupled to the supercontinuum source provided a spectrally tunable illumination source for making the multiple wavelength measurements. Alternatively, other means for generating light at desired wavelengths can be envisaged, e.g. separate lasers which by emit at a fixed of tunable wavelength.

In the embodiment shown, the polychromatic light beam from the AOTF is coupled to a single mode fibre e.g. with a core diameter of 4$\mu$m. In the embodiment shown, the light is collimated using a reflective collimator C1 e.g. having an effective focal length of 15 mm to a collimated beam of diameter 4 mm. Of course also other optical elements such as lenses, beam shapers, pinholes et cetera and can be used to provide a collimated beam of any desired diameter.

In the embodiment shown, the aperture A1 reduced the beam diameter to 2 mm. In the embodiment shown, a beam splitter (BS) is used to split the illumination and detection beams e.g. in a ratio of 90(T):10(R). In the embodiment shown, a telescope is used. For example, the telescope is made of lenses L1 (f =50 mm) and L2 (f =50 mm). In the embodiment shown, the beam is relayed on BS to the galvo mirror G1, which in turn is relayed on the galvo mirror G2 using the curved mirrors CM1 (e.g. f =100 mm) and CM2 (e.g. f =100 mm). Of course also other means for controlling beam position can be envisaged. The curved mirror CM3 (e.g. f =100 mm) and the lens L3 (e.g. f =75 mm) can be used to create a conjugate plane of G2 on the pupil plane for imaging.

For the experiments described below, a model eye E is used with an uncoated lens L4 (f =15.4 mm) having a curvature very similar to the cornea of the human eye. The sample S was placed in the focus of L4. It will be appreciated that a similar system can be used to image the retina of a real eye. The light is reflected and back-scattered by the sample and 90% of the reflected light passes through the beam splitter in the return path. In the embodiment shown, the dichroic mirrors DM1 (long pass 500 nm) and DM2 (long pass 594 nm) separate the reflected intensity into three channels. The lenses LD1, LD2 and LD3 (e.g. f =25 mm) focus the light on the core of the multimode fibres with a core diameter of 100 $\mu$m. In the embodiment shown, each multimode fibre is connected to a SPAD. Clean up filters CF1(488, bandwidth: 5 nm), CF2(525, bandwidth: 39 nm) can be placed in the blue and green channel to eliminate the crosstalk between the channels. The Red channel typically has no significant cross talk.

In the experiments performed, the imaging pixel rate for the phantoms was 30 kHz, although the maximum pixel rate of the system was 60 KHz. The size of a single image frame was 512 $\times$ 512 pixels. Thus, each image took 8.7 seconds to record. The essentially static nature of the phantoms posed no problems in terms of motion artefacts in the recorded image. Of course, also faster systems can be used particularly for imaging a real eye which may move. The key system parameters are detailed in Table 2. Using a relatively large core diameter of 100 $\mu$m in the detection eliminated the speckle noise in the final image at the expense of spatial resolution due to reduced confocality.

**Table 2.** System parameters of the SLO used in the experiments described herein

| System Parameter | Value |
| --- | --- |
| Illuminated pot size on the retinal plane (548 nm) | 13.7 $\mu$m (airy disk diameter) |
| maximum Field of view (FOV) | 33° in air |
| pixel Rate | 30 kHz |
| digital Resolution | 11.71 $\mu$m |

In the embodiment of the SLO design, we describe single-mode fibre illumination and multimode fibre detection. Hence the imaging may not be truly confocal, but quasi-confocal or sub-diffuse. It will be appreciated that quasi-confocal, sub-diffuse detection may reduce the central light reflex, a bright specular reflection from the centre of a blood vessel that is common to many fundus imaging modalities that do not use cross polarizers. When the central light reflex is present in an image, the intensity in the centre of the vessel is extracted by reconstructing the blood vessel profile using analytical models, such as a fourth order polynomial. This would otherwise add additional error to the blood vessel OD estimation and thereby, the saturation estimation.

In the following, measurements are described in a model eye using a retina mimicking phantom. The phantom fabrication and wavelength selection was as follows. Uniformly scattering phantoms were manufactured. First, a mixture of TiO$_2$ (Titanium(IV) oxide, Sigma Aldrich, mixture of rutile and anatase, nanopowder, less than 100 nm particle size, part no. 634662) and enhanced polarity and low viscosity Silicone oil (Sigma Aldrich, Poly (dimethylsiloxane), hydroxy-terminated, , part no. 481955) was made in a 1:9 mass ratio. Just before making the phantoms, the stock solution was sonicated at 132 KHz in an ultrasonic bath for 60 minutes. This results in a 10 w% TiO$_2$ stock solution. A small portion of which is then mixed with a suitable amount of silicone elastomer (Sylgard 184, Dow Corning) to get desired w% of

TiO$_2$[45] giving wavelength dependent reduced scattering coefficients $(\mu'_s(\lambda))$ similar to that expected for the human retina. Transparent silicone curing agent was added to the mixture in a 1:10 mass ratio. The mixture is then degassed in a vacuum chamber at 8 mbar pressure till all the air bubbles disappeared. Using mixtures with different w% of TiO$_2$, layered phantoms were made in a petri dish, each layer cured in a thermal oven at 80°C for 60 minutes. Thin metallic wires of different diameter thickness (90 $\mu$m and 140 $\mu$m) were embedded in the topmost scattering layer of the phantom while still in the viscous state and then cured. Although we use relatively large diameter blood vessels in the validation experiments as presented herein, this is not a limitation of the imaging technique as e.g. current SLO technology can image blood vessels smaller than 50 $\mu$m. In the current setup, a transparent layer of silicone without any TiO$_2$ particles was poured finally to provide mechanical stability to the phantom. After solidifying completely, the metallic wires were removed from the phantom. For our experiments, we used three layer phantoms (FIG 10A) with a transparent top layer,

a thin 200 $\mu$m layer with $\mu'_s = 1 \text{ mm}^{-1}$ with channels in the middle, and a bottom layer with $\mu'_s = 5 \text{ mm}^{-1}$.

FIG 10B shows an OCT B-scan (cross-section) of the phantom used for the measurements in the model eye. Two water-soluble dyes - Evans Blue (Sigma Aldrich B.V., The Netherlands) and red food colourant (Lebenmittelfarbe, Birkmanns Voedelkleurstof, Germany) were used to simulate the deoxyhaemoglobin and oxyhaemoglobin absorption, respectively. Using a suitable concentration of these dyes, an artificial isosbestic point was created at 548 nm as shown in Fig. 10C. Using a syringe, different calibrated proportions of the dyes were injected into the channels.

If [EB] is the concentration of Evans Blue and [FC] is the concentration of the red food colourant, we define a parameter $\zeta$ as

$$\textbf{Equation 25}$$
$$\varsigma = \frac{[FC]}{[FC]+[EB]}$$

The absorption coefficient of the mixture in this case becomes

$$\textbf{Equation 26}$$
$$\mu_a(\bar{x}_c, \lambda) = \upsilon \cdot \left( \varsigma \cdot \mu_a^{FC}(\lambda) + (1-\varsigma) \cdot \mu_a^{EB}(\lambda) \right)$$

where $\mu_a^{EB}(\lambda)$ and $\mu_a^{FC}(\lambda)$ are the absorption coefficients of Evans blue and the red food colourant, respectively and Eq. 13 now becomes,

$$\textbf{Equation 27}$$
$$\varsigma_{\lambda a|548} = \frac{D_C \cdot \mu_a^{EB}(\lambda_p) + \ln\left(1 + \rho \cdot \exp\left(-D_C \cdot \mu_a^i\right) - \rho_{\lambda p|548}\right)}{D_C \cdot \left(\mu_a^{EB}(\lambda_a) - \mu_a^{FC}(\lambda_a)\right)}$$

where $D_C$ is the channel diameter.

An analysis similar as described above was performed to find the wavelengths resulting in low errors when estimating the parameter $\zeta$. The result of the analysis is shown in FIG 11 for different "saturation - $\zeta$" levels. From FIG 11, we see a band centred around 502 nm and 610 nm wavelengths resulting in low saturation errors for $\zeta$ = 0.00 and $\zeta$ = 1.00, respectively. Due to the choice of the dichroic mirrors (DM1 and DM2 in Fig. 9) in the detection channel, 488 nm and 612 nm were chosen as the two 'anisosbestic' wavelengths to measure the saturation. Using a suitable ratio of [FC] and [EB], solutions with five different $\zeta$ (0.00, 0.25, 0.50, 0.75 and 1.00) were made and injected into the channels using a syringe. The phantoms were imaged simultaneously using the three different wavelengths, 488 nm (bandwidth, FWHM: 2 nm), 612 nm (bandwidth, FWHM: 3 nm) and 548 nm (isosbestic) (bandwidth, FWHM: 2 nm), and en *face* images were created for each wavelength. The number of reflected photons from TiO$_2$ and the dye-filled channel was estimated from the image and the parameter $\zeta$ was then calculated after estimating the ODs, $\rho$ and $D_C$ values from the images.

FIG 12 illustrates a typical intensity profile of a blood vessel (real or model). In one embodiment, the blood vessel diameter (D$_{ves}$) is determined from the intensity profile itself. For example, the diameter can be determined by the distance (in pixels) between the points with maximum slope magnitude along the blood vessel profile *v1* and *v2*. Of course also other recognizable points in the profile e.g. at a halfway or other fractional point between maximum and minimum intensities can be used, optionally with appropriate scaling. In another or further embodiment, the vessel diameter is determined by other means, e.g. by fitting the diameter as another parameter to multivariate data including three or more wavelengths.

In one embodiment, the intensity at the blood vessel location is estimated by the minima of the vessel profile. In some embodiments, e.g. in case there is a reflection artefact in the centre of the blood vessel, the location x$_b$ of point b and its intensity can be determined through extrapolation e.g. of polynomial fits of the vessel profile excluding the central vessel reflection artefact.

In one embodiment, the intensity of a tissue location is measured at one or more locations adjacent or near a selected blood vessel. In the embodiment shown, the intensity at the corresponding tissue location t is estimated using two points *t1* and *t2* e.g. by a (linear) approximation e.g. transverse to a direction of the vessel profile. Preferably, the tissue points represent locations where the light has not or negligibly interacted with the blood and are selected e.g. at a fixed number of pixels or distance from the vessel, e.g. in relation to b and/or *v1* and *v2*, respectively such that the locations *t* and *b* are affected by similar factors except the absorption by haemoglobin. For example, one or more of the tissue points such as *t1* and *t2,* can be selected at a distance depending on the vessel diameter e.g. multiplied by a factor such as $2 \times D_{ves}$ from on each side from *v1* and *v2*. In order to increase the accuracy of the estimated intensities (points t and b), the points can be averaged along multiple points in the tissue (avg t) and in the middle of the blood vessel (avg b)as shown in FIG 12. In one embodiment, as shown, the intensity $I(x_b, \lambda)$ is obtained by averaging the intensities of the points b along the valley (along with the y dimension - denoted by dotted lines). In one embodiment, as shown, the point t and thereby $I(x_t, \lambda)$ is obtained by averaging the points in an area as shown in FIG 12.

FIGs 13A - C illustrate example reflectance experiments for 100 % food colouring ($\zeta$ = 1.00) showing high absorption at 488 nm (Fig. 13A). Figure 13D - Fig. 13F shows the reflectance measurements for 0 % food colouring ($\zeta$ = 0.00). Measurement points were chosen along the centre of the channel and in the tissue ($TiO_2$ layer in this case) to estimate the reflected light intensity ($I_b$ and $I_t$). In order to bring the standard error on the mean intensity in both the tissue and along the blood vessels to desired values of < 1 %, up to 400 points were averaged in the tissue location and 40 points were averaged in the blood vessel location. The intensity profiles along the lines indicated in FIGs 13D - 13F are plotted in FIG. 13G. The dip in the intensity profile due to the absorption of blood will be noted in FIG 13G. It will be appreciated that e.g. due to the sub-diffuse nature of the reflectance measurements, caused by using a large (100nm) pinhole, there appear to be no reflection artefacts in the middle of the channel.

FIG 14 illustrates a comparison between calibrated (Cal) and estimated (Est) $\zeta$ values. The o's denote the points where the $\zeta$ was calculated from the 488 nm, 548 nm pair whereas the x's denote the points where the 548 nm, 612 nm pair was used to calculate $\zeta$. Except for $\zeta$ = 0.5, all the $\zeta$ values have a $\Delta\zeta \leq 0.1$. When using the 488-548 nm wavelength pair to estimate $\zeta$ values above 0.5, the error in $\zeta$ became as high as 0.9, demonstrating the rationale mentioned in the previous section that two anisosbestic wavelengths are desirable in order to cover the whole saturation regime [0 1].

FIG 15 illustrates a grayscale map of the saturation $\zeta$ overlaid on the corresponding reflectance image from the SLO. As explained previously, the noise in the images may propagates into the saturation error. In order to bring the saturation error to desired levels of e.g. less than 10%, a number of points along the blood vessel and the tissue location may be averaged. While this is relatively easy in our experiments due to the homogeneous nature of the phantom, an *in vivo* image contains structural information and choosing a large number of points might lead to an incorrect estimation of the intensity in the absence of a blood vessel. In order to overcome this, a smaller number of points around the blood vessel may be averaged, which may require multiple images to be produced within a short amount of time. Fundus cameras are based on snapshot imaging and it might not always be possible to acquire multiple images continuously without causing discomfort to the subject or exceeding the safety limit for radiant exposure. Although our current SLO images at relatively slow speeds, significant improvements in speed can be achieved by line scanning instead of a spot scanning approach, with a reduced signal-to-noise ratio. Current commercial SLO technology has an imaging speed of about 30 Hz, which facilitates the averaging of multiple consecutive images to accomplish noise reduction.

The effective path length (<L>) that photons travel through a tissue volume before being collected may depend on the illumination and collection geometry, and can also be a function of the optical properties of the tissue and can thus be wavelength dependent. In some embodiments, it is assumed that the effective path length for the two wavelengths used to calculate the factor $\rho$ (Eq. 12) is almost the same. This assumption may e.g. be valid for a sufficiently small change in wavelengths, especially in the blue-green wavelength range (450-600 nm) where absorption remains of the same order of magnitude; scattering varies slowly with wavelength (roughly as 1/lambda) for these wavelengths as well.

For the phantoms that we fabricated, scattering was a slowly varying function of wavelength, similar to tissue, and the absorption coefficients of the dyes in the channels were of the same order of magnitude as the absorption coefficients of oxy- and deoxyhaemoglobin. For our phantoms, we have shown to accurately recover the "saturation' in the channels, suggesting that there were no significant changes in the path length due to scattering and absorption in a tissue-mimicking phantom with relevant scattering and absorbing properties. Additionally, pigments which are present in the retina and absorption background due to choroid blood can affect the recorded intensity. However, by virtue of the Eqs. 3, 6 and 12, these additional background effects are cancelled out while making a ratiometric measurement of the ODs. It will be noted that insufficient removal of the system reflections from the recorded intensities may result in a wrong estimation of the ODs and hence the saturation. Therefore, it is recommended in some embodiments that all the internal system reflections are subtracted e.g. by taking a reference measurement without any sample.

The present study represents a new development in choosing an optimal wavelengths for retinal oximetry taking into account the haemoglobin packaging effect. This is different than simply choosing one isosbestic wavelength and another non-isosbestic wavelength where $\left| \mu_a^{HbO_2}(\lambda) - \mu_a^{Hb}(\lambda) \right|$ is maximum, e.g. 570nm (isosbestic) and 600 nm.

While this wavelength combination might give sufficiently accurate saturation estimation in arteries, our analysis shows that they may not be reliable for low saturation values of S < 60%, which might occur due to various pathological conditions. In some embodiments, the present modified oximetry equations may provide e.g. the 460, 548 (isosbestic) and 594 nm triad as one possible combinations for oximetry. Although this choice spans a relatively broad wavelength range (~130 nm), the absorption coefficients and scattering coefficients in this wavelength range are similar to those we have used in our phantoms, suggesting that the effective path lengths differences within this wavelength range are of minor importance. The accuracy of saturation estimation can be potentially further improved by including more wavelengths.

In interpreting the appended claims, it should be understood that the word "comprising" does not exclude the presence of other elements or acts than those listed in a given claim; the word "a" or "an" preceding an element does not exclude the presence of a plurality of such elements; the scope of the current invention is defined by the claims.

**Claims**

1. A method for measuring retinal oxygenation (S) comprising

   - illuminating a retinal tissue having blood vessels by light essentially consisting of a selection of two or more discrete wavelengths ($\lambda_1, \lambda_2$);
   - collecting measurements of a plurality of backscattered reflection intensities ( $I(x_b, \lambda_1)$, $I(x_t, \lambda_1)$, $I(x_b, \lambda_2)$, $I(x_t, \lambda_2)$ ), wherein the measurements are spectrally resolved for the selection of discrete wavelengths and position-resolved for at least a blood vessel location ($x_b$) coinciding with a selected blood vessel and a tissue location ($x_t$) coinciding with the retinal tissue without blood vessel, wherein a size ($D_{ves}$) of the selected blood vessel is determined based on the position resolved measurements of the plurality of backscattered reflection intensities; and
   - calculating, by a processor, the oxygenation (S) based on the measurements, wherein the calculation includes an analytical model describing a dependence of the intensities on the oxygenation (S) of blood in the selected blood vessel and the determined size ($D_{ves}$) of the selected blood vessel, wherein the analytical model includes calculating an error ($\Delta S$) in the calculated oxygenation (S), wherein the calculation of the error ($\Delta S$) includes the determined size ($D_{ves}$) of the selected blood vessel, wherein the number of collected measurements is based on the determined size ($D_{ves}$) of the selected blood vessel such that the collecting of measurements is continued until the calculated error ($\Delta S$), dependent on the determined size ($D_{ves}$), is below a predetermined threshold error.

2. The method according to claim 1, wherein the number of collected measurements for calculating the oxygenation is relatively small for a relatively large size ($D_{ves}$) of the selected blood vessel, and the number of collected measurements for calculating the oxygenation is relatively high for a relatively small size ($D_{ves}$) of the selected blood vessel.

3. The method according to any of the preceding claims, wherein the analytical model includes an effective absorption coefficient ($\mu_a^{eff}$) of the blood vessel at the selection of wavelengths calculated as function of

   - predetermined absorption coefficients of homogenously distributed oxy- and deoxyhaemoglobin (HbO$_2$, Hb) at the selected wavelengths ($\lambda$) weighted according to the oxygenation (S) defined as a fraction of one of the oxy- and deoxyhaemoglobin (HbO$_2$, Hb) over a total hemoglobin (HbO$_2$+Hb); and
   - a correction factor ($C_{corr}$) depending on a size ($D_{ves}$) of the selected blood vessel to correct for a pigment packaging effect of absorbing haemoglobin molecules not being homogeneously distributed throughout the retinal tissue but contained in a discrete package of the selected blood vessel.

4. The method according to any of the preceding claims, wherein the analytical model includes a term proportional with

   - [ 1 - exp( - $D_{ves} \cdot \mu_{a,ves}$ ) ], where
   o $D_{ves}$ is proportional to a diameter the selected blood vessel, and $\mu_{a,ves}$ is proportional to an absorption coefficient of blood in the blood vessel according to a linear combination of oxy- and deoxyhaemoglobin (HbO$_2$, Hb) spectra depending on the oxygenation (S).

5. The method according to any of the preceding claims, wherein the selection of wavelengths includes at least one isosbestic wavelength ($\lambda_i$) where oxy- and deoxyhaemoglobin (HbO$_2$,Hb) have the same absorption coefficient ($\Delta\mu_a$=0) and at least one anisosbestic wavelength ($\lambda_a$) where oxy- and deoxyhaemoglobin (HbO$_2$,Hb) have a different absorption coefficient, wherein the anisosbestic wavelength is in a range of 596 $\pm$ 7 nm.

6. The method according to any of the preceding claims, wherein the selection of wavelengths includes a first anisosbestic wavelength ($\lambda_{a,1}$) where oxyhaemoglobin (HbO$_2$) has a lower absorption coefficient ($\Delta\mu_a<0$) than deoxyhaemoglobin (Hb), and a second anisosbestic wavelength ($\lambda_{a,2}$) where oxyhaemoglobin (HbO$_2$) has a higher absorption coefficient ($\Delta\mu_a>0$) than deoxyhaemoglobin (Hb).

7. The method according to the preceding claim, wherein the first anisosbestic wavelength ($\lambda_{a,1}$) is above 586 nm and the second anisosbestic wavelength($\lambda_{a,1}$) is below 506 nm, or between 522 - 548 nm, or between 569 - 586 nm.

8. The method according to the preceding claim, wherein the first anisosbestic wavelength ($\lambda_{a,1}$) is in a range of 596 $\pm$ 7 nm.

9. The method according to the preceding claim, wherein the second anisosbestic wavelength ($\lambda_{a,2}$) is in a range of 466 $\pm$ 16 nm or 539 $\pm$ 8 nm or 578 $\pm$ 7 nm.

10. The method according to any of the preceding claims, wherein the selection of wavelengths includes at least one isosbestic wavelength ($\lambda_i$) where oxy- and deoxyhaemoglobin (HbO$_2$,Hb) have the same absorption coefficient ($\Delta\mu_a=0$) and at least two anisosbestic wavelengths ($\lambda_{a,1}$,$\lambda_{a,2}$) where oxy- and deoxyhaemoglobin (HbO$_2$,Hb) have a different absorption coefficient.

11. The method according to the preceding claim, wherein the second anisosbestic wavelength ($\lambda_{a,2}$) is in a range of 466 $\pm$ 16 nm.

12. The method according to the preceding claim, wherein the isosbestic wavelength ($\lambda_i$) is 506 nm, 522 nm, or 586 nm.

13. A system for measuring retinal oxygenation (S) comprising

- a light source configured to generate light essentially consisting of a selection of two or more discrete wavelengths ($\lambda_1$,$\lambda_2$)
- projection optics configured to receive the light from the light source and illuminate a retinal tissue with blood vessels by the light, wherein the projection are configured to position-resolve at least a blood vessel location ($x_b$) coinciding with a selected blood vessel and a tissue location ($x_t$) coinciding with the retinal tissue without blood vessel;
- collection optics configured to receive and measure backscattered reflection intensities ( $I(x_b,A_i)$, $I(x_t,\lambda_1)$, $I(x_b,\lambda_2)$, $1(x_t,\lambda_2)$ ) of the light reflected of the retinal tissue with blood vessels, wherein the collection optics are configured to spectrally resolve the light for the selection of discrete wavelengths; and
- a processor programmed to

determine a size ($D_{ves}$) of the selected blood vessel based on the position resolved measurements of the plurality of backscattered reflection intensities,
control the number of collected measurements based on the determined size ($D_{ves}$) of the selected blood vessel, and
calculate the oxygenation (S) based on the measured intensities, wherein the calculation includes an analytical model describing a dependence of the intensities on the oxygenation (S) of blood in the selected blood vessel and the determined size ($D_{ves}$) of the selected blood vessel, wherein the analytical model includes calculating an error ($\Delta S$) in the calculated oxygenation (S), wherein the calculation of the error ($\Delta S$) includes the determined size ($D_{ves}$) of the selected blood vessel, wherein the collecting of measurements is controlled to continue until the calculated error ($\Delta S$), dependent on the determined size ($D_{ves}$), is below a predetermined threshold error.

14. A computer readable medium comprising software instructions, which when executed by a processor of the system of claim 13, cause the system to perform the method according to any of claims 1 - 12.

**Patentansprüche**

1. Verfahren zur Messung der Netzhautoxygenierung (S) umfassend

- Beleuchtung eines Netzhautgewebes, das Blutgefäße aufweist, durch Licht, das im Wesentlichen aus einer Auswahl von zwei oder mehr diskreten Wellenlängen ($\lambda_1, \lambda_2$) besteht;
- Erfassen von Messungen mehrerer rückgestreuter Reflexionsintensitäten ($I(x_b, \lambda_1)$, $1(x_t, \lambda_1)$, $I(x_b, \lambda_2)$, $I(x_t, \lambda_2)$), wobei die Messungen für die Auswahl diskreter Wellenlängen spektral aufgelöst und für wenigstens eine Blutgefäßposition ($x_b$), die mit einem ausgewählten Blutgefäß übereinstimmt, und eine Gewebeposition ($x_t$), die mit dem Netzhautgewebe ohne Blutgefäß übereinstimmt, positionsaufgelöst sind, wobei eine Größe($D_{ves}$) des ausgewählten Blutgefäßes auf der Grundlage der positionsaufgelösten Messungen mehrerer rückgestreuter Reflexionsintensitäten bestimmt wird; und
- Berechnen der Oxygenierung (S) durch einen Prozessor auf der Grundlage der Messungen, wobei die Berechnung ein analytisches Modell einschließt, das eine Abhängigkeit der Intensitäten von der Oxygenierung (S) des Blutes in dem ausgewählten Blutgefäß und der bestimmten Größe ($D_{ves}$) des ausgewählten Blutgefäßes beschreibt, wobei das analytische Modell das Berechnen eines Fehlers ($\Delta S$) in der berechneten Oxygenierung (S) einschließt, wobei die Berechnung des Fehlers ($\Delta S$) die bestimmte Größe ($D_{ves}$) des ausgewählten Blutgefäßes einschließt, wobei die Anzahl der erfassten Messungen auf der bestimmten Größe ($D_{ves}$) des ausgewählten Blutgefäßes basiert, so dass das Erfassen von Messungen fortgesetzt wird, bis der berechnete Fehler ($\Delta S$), der von der bestimmten Größe ($D_{ves}$) abhängt, unter einem vorbestimmten Schwellenfehler liegt.

2. Verfahren nach Anspruch 1, wobei die Anzahl der erfassten Messungen zur Berechnung der Oxygenierung bei einer relativ großen Größe ($D_{ves}$) des ausgewählten Blutgefäßes relativ gering ist und die Anzahl der erfassten Messungen zur Berechnung der Oxygenierung bei einer relativ kleinen Größe ($D_{ves}$) des ausgewählten Blutgefäßes relativ hoch ist.

3. Verfahren nach einem der vorhergehenden Ansprüche, wobei das analytische Modell einen effektiven Absorptionskoeffizienten ($\mu_a^{eff}$ des Blutgefäßes bei der Auswahl der Wellenlängen einschließt, als Funktion von

- vorbestimmten Absorptionskoeffizienten von homogen verteiltem Oxy- und Desoxyhämoglobin ($HbO_2$, Hb) bei den ausgewählten Wellenlängen ($\lambda$), gewichtet nach der Oxygenierung (S), definiert als ein Anteil des Oxy- oder Desoxyhämoglobins ($HbO_2$, Hb) am Gesamthämoglobin ($HbO2^+Hb$); und
- einem Korrekturfaktor ($C_{corr}$), der von der Größe ($D_{ves}$) des ausgewählten Blutgefäßes abhängt, um einen Pigmentverpackungseffekt des Absorbierens der Hämoglobinmoleküle, die nicht über das gesamte Netzhautgewebe verteilt, sondern in einem diskreten Paket des ausgewählten Blutgefäßes enthalten sind, zu korrigieren.

4. Verfahren nach einem der vorhergehenden Ansprüche, wobei das analytische Modell eine Größe einschließt, die proportional ist zu

- [ 1 - exp( - Dves · $\mu_{a,ves}$) ], wobei
o $D_{ves}$ proportional zu einem Durchmesser des ausgewählten Blutgefäßes ist, und $\mu_{a,ves}$ proportional zu einem Absorptionskoeffizienten des Blutes im Blutgefäß gemäß einer linearen Kombination von Oxy- und Deoxyhämoglobin- ($HbO_2$, Hb) Spektren in Abhängigkeit von der Oxygenierung (S) ist.

5. Verfahren nach einem der vorhergehenden Ansprüche, wobei die Auswahl der Wellenlängen wenigstens eine isosbestische Wellenlänge ($\lambda_i$) einschließt, wobei Oxy- und Deoxyhämoglobin ($HbO_2$, Hb) den gleichen Absorptionskoeffizienten ($\Delta \mu_a = 0$) und wenigstens eine anisosbestische Wellenlänge ($\lambda_a$) aufweisen, wobei Oxy- und Deoxyhämoglobin ($HbO_2$, Hb) einen unterschiedlichen Absorptionskoeffizienten aufweisen, wobei die anisosbestische Wellenlänge in einem Bereich von 596 $\pm$ 7 nm liegt.

6. Verfahren nach einem der vorhergehenden Ansprüche, wobei die Auswahl der Wellenlängen eine erste anisosbestische Wellenlänge ($\lambda_{a,1}$) einschließt, wobei Oxyhämoglobin ($HbO_2$) einen niedrigeren Absorptionskoeffizienten ($\Delta \mu_a < 0$) als Deoxyhämoglobin (Hb) aufweist, und eine zweite anisosbestische Wellenlänge ($\lambda_{a,2}$), wobei Oxyhämoglobin ($HbO_2$) einen höheren Absorptionskoeffizienten ($\Delta \mu_a > 0$) als Deoxyhämoglobin (Hb) aufweist.

7. Verfahren nach dem vorhergehenden Anspruch, wobei die erste anisosbestische Wellenlänge ($\lambda_{a,1}$) über 586 nm und die zweite anisosbestische Wellenlänge($\lambda_{a,1}$) unter 506 nm oder zwischen 522 - 548 nm oder zwischen 569 - 586 nm liegt.

8. Verfahren nach dem vorhergehenden Anspruch, wobei die erste anisosbestische Wellenlänge ($\lambda_{a,1}$) im Bereich von 596 $\pm$ 7 nm liegt.

**9.** Verfahren nach dem vorhergehenden Anspruch, wobei die zweite anisosbestische Wellenlänge ($\lambda_{a,2}$) im Bereich von 466 $\pm$ 16 nm oder 539 $\pm$ 8 nm oder 578 $\pm$ 7 nm liegt.

**10.** Verfahren nach einem der vorhergehenden Ansprüche, wobei die Auswahl der Wellenlängen wenigstens eine isosbestische Wellenlänge ($\lambda_i$) einschließt, wobei Oxy- und Deoxyhämoglobin (HbO$_2$,Hb) den gleichen Absorptionskoeffizienten ($\Delta\mu_a$=0) und wenigstens zwei anisosbestische Wellenlängen ($\lambda_{a,1}$,$\lambda_{a,2}$) aufweisen, wobei Oxy- und Deoxyhämoglobin (HbO$_2$,Hb) einen unterschiedlichen Absorptionskoeffizienten aufweisen.

**11.** Verfahren nach dem vorhergehenden Anspruch, wobei die zweite anisosbestische Wellenlänge ($\lambda_{a,2}$) im Bereich von 466 $\pm$ 16 nm liegt.

**12.** Verfahren nach dem vorhergehenden Anspruch, wobei die isosbestische Wellenlänge ($\lambda_i$) 506 nm, 522 nm oder 586 nm beträgt.

**13.** System zur Messung der Netzhautoxygenierung (S), umfassend

- eine Lichtquelle, die dazu eingerichtet ist, Licht zu erzeugen, das im Wesentlichen aus einer Auswahl von zwei oder mehr diskreten Wellenlängen ($\lambda_1$,$\lambda_2$) besteht
- Projektionsoptik, die dazu eingerichtet ist, das Licht von der Lichtquelle zu empfangen und durch das Licht ein Netzhautgewebe mit Blutgefäßen zu beleuchten, wobei die Projektion zur Positionsauflösung wenigstens einer mit einem ausgewählten Blutgefäß übereinstimmenden Blutgefäßposition ($x_b$) und einer mit dem Netzhautgewebe übereinstimmenden Gewebeposition ($x_t$) ohne Blutgefäß eingerichtet ist;
- Erfassungsoptik, die dazu eingerichtet sind, rückgestreute Reflexionsintensitäten ($I(x_b,\lambda_1)$, $I(x_t,\lambda_1)$, $I(x_b,\lambda_2)$, $I(x_t,\lambda_2)$) des vom Netzhautgewebe mit Blutgefäßen reflektierten Lichts zu empfangen und zu messen, wobei die Erfassungsoptik dazu eingerichtet ist, das Licht für die Auswahl diskreter Wellenlängen spektral aufzulösen; und
- einen Prozessor, der so programmiert ist, dass er

eine Größe ($D_{ves}$) des ausgewählten Blutgefäßes auf der Grundlage der positionsaufgelösten Messungen mehrerer rückgestreuter Reflexionsintensitäten bestimmt, die Anzahl der erfassten Messungen auf der Grundlage der bestimmten Größe ($D_{ves}$) des ausgewählten Blutgefäßes steuert und die Oxygenierung (S) auf der Grundlage der gemessenen Intensitäten berechnet,
wobei die Berechnung ein analytisches Modell einschließt, das eine Abhängigkeit der Intensitäten der Oxygenierung (S) des Blutes im ausgewählten Blutgefäß und der bestimmten Größe ($D_{ves}$) des ausgewählten Blutgefäßes beschreibt, wobei das analytische Modell das Berechnen eines Fehlers ($\Delta S$) in der berechneten Oxygenierung (S) einschließt, wobei die Berechnung des Fehlers ($\Delta S$) die bestimmte Größe ($D_{ves}$) des ausgewählten Blutgefäßes einschließt, wobei das Erfassen gesteuert wird, um fortzufahren, bis der berechnete Fehler ($\Delta S$), abhängig von der bestimmten Größe ($D_{ves}$), unter einem vorbestimmten Schwellenfehler liegt.

**14.** Computerlesbares Medium, umfassend Softwareanweisungen, die, wenn sie von einem Prozessor des Systems nach Anspruch 13 ausgeführt werden, bewirken, dass das System das Verfahren nach einem der Ansprüche 1 bis 12 durchführt.

**Revendications**

**1.** Procédé de mesure de l'oxygénation rétinienne (S) comprenant les étapes consistant à :

- éclairer un tissu rétinien ayant des vaisseaux sanguins par la lumière consistant essentiellement en une sélection de deux ou plusieurs longueurs d'onde discrètes ($\lambda_l$, $\lambda_2$);
- collecter des mesures d'une pluralité d'intensités de réflexion rétrodiffusées ($I(x_b,\lambda_1)$, $I(x_t,\lambda_1)$, $I(x_b,\lambda_2)$, $I(x_t,\lambda_2)$, dans lequel les mesures sont résolues spectralement pour la sélection de longueurs d'onde discrètes et résolues en position pour au moins un emplacement de vaisseau sanguin ($x_b$) coïncidant avec un vaisseau sanguin sélectionné et un emplacement tissulaire ($x_t$) coïncidant avec le tissu rétinien sans vaisseau sanguin, dans lequel une taille ($D_{ves}$) du vaisseau sanguin sélectionné est déterminée sur la base des mesures résolues pour la position de la pluralité des intensités de réflexion rétrodiffusées ; et
- calculer, par un processeur, l'oxygénation (S) sur la base des mesures, dans lequel le calcul comprend un modèle analytique décrivant une dépendance des intensités sur l'oxygénation (S) du sang dans le vaisseau sanguin sélectionné et la taille déterminée ($D_{ves}$) du vaisseau sanguin sélectionné, dans lequel le modèle analytique comprend le calcul d'une erreur ($\Delta S$) dans l'oxygénation calculée (S), dans lequel le calcul de l'erreur ($\Delta S$) inclut la taille déterminée ($D_{ves}$) du vaisseau sanguin sélectionné, dans lequel le nombre de mesures collectées est basé sur la taille déterminée ($D_{ves}$) du vaisseau sanguin sélectionné de sorte que la collecte des mesures se poursuit jusqu'à ce que l'erreur calculée ($\Delta S$), en fonction de la taille déterminée ($D_{ves}$), est inférieur à une erreur de seuil prédéterminée.

2. Procédé selon la revendication 1, dans lequel le nombre de mesures collectées pour le calcul de l'oxygénation est relativement faible pour une taille relativement grande ($D_{ves}$) du vaisseau sanguin sélectionné, et le nombre de mesures collectées pour le calcul de l'oxygénation est relativement élevé pour une taille relativement petite ($D_{ves}$) du vaisseau sanguin sélectionné.

3. Procédé selon l'une quelconque des revendications précédentes, dans lequel le modèle analytique comprend un coefficient d'absorption efficace ($\mu_a^{eff}$) du vaisseau sanguin au niveau de la sélection des longueurs d'onde, calculé en fonction

- des coefficients d'absorption prédéterminés d'oxy- et de désoxyhémoglobine distribuée de manière homogène ($HbO_2$, Hb) aux longueurs d'onde sélectionnées ($\lambda$) pondérées en fonction de l'oxygénation (S) définie comme une fraction de l'oxy- et de la désoxyhémoglobine ($HbO_2$, Hb) sur une hémoglobine totale ($HbO_2$+Hb) ; et
- un facteur de correction ($C_{corr}$) dépendant d'une taille ($D_{ves}$) du vaisseau sanguin sélectionné pour corriger un effet de regroupement de pigmentation de l'absorption de molécules d'hémoglobine qui ne sont pas réparties de manière homogène dans tout le tissu rétinien mais qui sont contenues dans un regroupement discret du vaisseau sanguin sélectionné.

4. Procédé selon l'une quelconque des revendications précédentes, dans lequel le modèle analytique comprend un terme proportionnel à

$$[\, 1\text{-} \exp(\, \text{-} D_{ves} \bullet \mu_{a,ves})\, ]$$

où

- $D_{ves}$ est proportionnel à un diamètre du vaisseau sanguin sélectionné, et ($\mu_{a,ves}$ est proportionnel à un coefficient d'absorption du sang dans le vaisseau sanguin selon une combinaison linéaire des spectres d'oxy- et désoxyhémoglobine ($HbO_2$, Hb) en fonction de l'oxygénation (S).

5. Procédé selon l'une quelconque des revendications précédentes, dans lequel la sélection des longueurs d'onde comprend au moins une longueur d'onde isosbestique ($\lambda_i$) où l'oxy- et la désoxyhémoglobine ($HbO_2$, Hb) ont le même coefficient d'absorption ($\Delta\mu_a=0$) et au moins une longueur d'onde anisosbétique ($\lambda_a$) où l'oxy- et désoxyhémoglobine ($HbO_2$, Hb) ont un coefficient d'absorption différent, dans lequel la longueur d'onde anisosbestique est comprise entre 596 $\pm$ 7 nm.

6. Procédé selon l'une quelconque des revendications précédentes, dans lequel la sélection des longueurs d'onde comprend une première longueur d'onde anisosbétique ($\lambda_{a,1}$) où l'oxyhémoglobine ($HbO_2$) a un coefficient d'absorption inférieur ($\Delta\mu_a<0$) à celui de la désoxyhémoglobine (Hb), et une deuxième longueur d'onde anisosbésique ($\lambda_{a,2}$) où l'oxyhémoglobine ($HbO_2$) a un coefficient d'absorption plus élevé ($\Delta\mu_a>0$) que la désoxyhémoglobine (Hb).

7. Procédé selon la revendication précédente, dans lequel la première longueur d'onde anisosbétique ($\lambda_{a,1}$) est supérieure à 586 nm et la deuxième longueur d'onde anisosbéstique ($\lambda_{a,1}$) est inférieure à 506 nm, ou comprise entre 522 et 548 nm, ou entre 569 et 586 nm.

8. Procédé selon la revendication précédente, dans lequel la première longueur d'onde anisosbéstique ($\lambda_{a,l}$) est comprise dans une plage de 596 $\pm$ 7 nm.

9. Procédé selon la revendication précédente, dans lequel la deuxième longueur d'onde anisosbétique ($\lambda a,2$) est comprise dans une plage de 466 $\pm$ 16 nm ou 539 $\pm$ 8 nm ou 578 $\pm$ 7 nm.

**10.** Procédé selon l'une quelconque des revendications précédentes, dans lequel la sélection des longueurs d'onde comprend au moins une longueur d'onde isosbestique ($\lambda i$) où l'oxy et le désoxyhémoglobine ($HbO_2$, Hb) ont le même coefficient d'absorption ($\Delta(\mu_a=0)$) et au moins deux longueurs d'onde anisosbéstiques ($\lambda_{a,1}$, $\lambda_{a,2}$) où l'oxy- et désoxyhémoglobine ($HbO_2$, Hb) ont un coefficient d'absorption différent.

**11.** Procédé selon la revendication précédente, dans lequel la deuxième longueur d'onde anisosbestique ($\lambda_{a,2}$) est comprise dans la plage de $466 \pm 16$ nm.

**12.** Procédé selon la revendication précédente, dans lequel la longueur d'onde isosbestique ($\lambda_i$) est de 506 nm, 522 nm, ou 586 nm.

**13.** Système de mesure de l'oxygénation rétinienne (S) comprenant

 - une source lumineuse configurée pour générer de la lumière consistant essentiellement en une sélection de deux ou plusieurs longueurs d'onde discrètes ($\lambda_l$, $\lambda_2$)
 - une optique de projection configurée pour recevoir la lumière de la source lumineuse et éclairer un tissu rétinien avec des vaisseaux sanguins par la lumière, dans lequel la projection est configurée pour résoudre en position au moins un emplacement de vaisseau sanguin ($x_b$) coïncidant avec un vaisseau sanguin sélectionné et un emplacement tissulaire ($x_t$) coïncidant avec le tissu rétinien sans vaisseau sanguin ;
 - une optique de collecte configurée pour recevoir et mesurer les intensités de réflexion rétrodiffusées ($I(x_b,\lambda_1)$, $I(x_t,\lambda_1)$, $I(x_b,\lambda_2)$, $I(x_t,\lambda_2)$) de la lumière réfléchie par le tissu rétinien avec les vaisseaux sanguins, dans lequel l'optique de collecte est configurée pour résoudre spectralement la lumière pour le sélection de longueurs d'onde discrètes ; et
 un processeur programmé pour :

   déterminer une taille ($D_{ves}$) du vaisseau sanguin sélectionné en fonction de la position résolue des mesures de la pluralité des intensités de réflexion rétrodiffusées,
   contrôler le nombre de mesures collectées en fonction de la taille déterminée ($D_{ves}$) du vaisseau sanguin sélectionné, et
   calculer l'oxygénation (S) en fonction des intensités mesurées,
   dans lequel le calcul comprend un modèle analytique décrivant une dépendance des intensités sur l'oxygénation (S) du sang dans le vaisseau sanguin sélectionné et la taille déterminée ($D_{ves}$) du vaisseau sanguin sélectionné, dans lequel le modèle analytique comprend le calcul d'une erreur ($\Delta S$) dans l'oxygénation calculée (S), dans lequel le calcul de l'erreur ($\Delta S$) inclut la taille déterminée ($D_{ves}$) du vaisseau sanguin sélectionné, dans lequel la collecte des mesures est contrôlée pour se poursuivre jusqu'à ce que l'erreur calculée ($\Delta S$), en fonction de la taille déterminée ($D_{ves}$), soit inférieure à une erreur seuil prédéterminée.

**14.** Support lisible par ordinateur comprenant des instructions logicielles qui, lorsqu'elles sont exécutées par un processeur du système de la revendication 13, amènent le système à exécuter le procédé selon l'une quelconque des revendications 1 à 12.

FIG 1A

FIG 1B

FIG 2

FIG 3

FIG 4A

FIG 4B

FIG 5

FIG 6

FIG 7

FIG 8

FIG 9

FIG 10A

FIG 10B

FIG 10C

FIG 11

FIG 12

FIG 13

FIG 14

FIG 15

FIG 16

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5776060 A

**Non-patent literature cited in the description**

- **FINLAY et al.** *Opt. Lett.,* 2004, vol. 29, 965-967
- **AMELINK.** *Opt. Lett.,* 2009, vol. 34, 1525-1527
- **RAJARAM et al.** *Lasers Surg. Med.,* 2010, vol. 42, 680-688